# FASCICULE DE BREVET EUROPEEN

(11) **EP 2 054 165 B1**
(45) Date de publication et mention de la délivrance du brevet: **30.03.2011**
(21) Numéro de dépôt: 07802572.3
(22) Date de dépôt: 10.08.2007
(51) Int. Cl.: B05D 5/00, B05D 1/18, A61K 8/11, A61K 31/66, A61L 27/32, A61K 49/18, A61K 31/6615

(54) **PROCEDE DE RECOUVREMENT DE SURFACES METALLIQUES OU MINERALES PAR DES COUCHES DE COMPOSES GEM-BISPHOSPHONIQUES ET LEURS UTILISATIONS**
VERFAHREN ZUM BEDECKEN VON FLÄCHEN AUS METALL ODER ANORGANISCHEN MONOSCHICHTEN MIT GEM-BISPHONSÄUREVERBINDUNGEN UND VERWENDUNGEN DAVON
METHOD OF COVERING SELF-ASSEMBLED METAL OR INORGANIC SURFACES WITH GEM-BISPHOSPHONIC COMPOUNDS AND USES THEREOF

(30) Priorité: 10.08.2006 FR 0607245
(43) Date de publication de la demande: 06.05.2009
(73) Titulaire: Surfactis Technologies, 49100 Angers (FR)
(72) Inventeur: PORTET, David, 49125 Briollay (FR); LECOLLINET, Grégory, 49100 Angers (FR); HINDRE, François, 35700 Rennes (FR); BEJANIN, Stéphane, 75005 Paris (FR); CHAPPARD, Daniel, 49100 Angers (FR); DENIZOT, Benoit, 74130 Bonneville (FR)
(74) Mandataire: Warcoin, Jacques
(86) Numéro de dépôt international: PCT/EP2007/058320
(87) Numéro de publication internationale: WO 2008/017721

(56) Documents cités:
- EP-A- 1 197 213
- FR-A- 2 848 850
- US-B1- 6 299 983

## Description

La présente invention a pour objet un procédé de recouvrement d'un substrat métallique ou minéral par une couche moléculaire de fonctionnalisation formée à partir de composés gem-bisphosphoniques.

La présente invention a également pour objet le substrat recouvert susceptible d'être obtenu à partir de ce procédé, les diverses utilisations de ce substrat en fonction de la nature des composés gem-bisphosphoniques (BP) greffés sur la surface du substrat, des composés gem-bisphosphoniques permettant la mise en oeuvre du procédé de recouvrement, et les diverses utilisations de ces composés gem-bisphosphoniques.

Le recouvrement de matériaux industriels de type métallique ou minéral est souvent envisagé sous plusieurs formes.

Tout d'abord, par un recouvrement de surface par un film plus ou moins épais (due quelques nanomètres à quelques millimètres) de polymères naturels ou synthétiques. La formation du film avec augmentation de la résistance mécanique dépend, suivant les cas, d'une réaction chimique de polymérisation (huiles siccatives), d'une évaporation du solvant ou d'une désolvatation des chaînes polymériques avec enchevêtrement (peintures dites en émulsion).

Bien que cette manière de faire puisse conduire à des revêtements de belle allure esthétique, ces revêtements présentent les inconvénients suivants:
- l'épaisseur de ces films exclut leur utilisation au sein de structure nano et microscopiques ;
- la tenue mécanique est limitée par la résistance du film : par exemple, le poinçonnage superficiel par des pièces ou des outils arrivant au contact qui progressivement provoque des irrégularités de surface qui rendent la surface difficile à nettoyer. Ceci explique que dans les industries pharmaceutiques et agro-alimentaires, le matériau préféré reste l'acier inoxydable nu qui résiste mieux aux contraintes mécaniques, chimiques et thermiques ;
- la tenue en température est souvent limitée par la fusion superficielle du film, ou par sa dégradation chimique : par exemple pour les ustensiles de cuisine, la mise au point de revêtements spéciaux de types perfluorés a permis la mise au point de tels instruments anti-adhésifs (vis à vis des aliments) et anti-salissure. De même, la résistance aux agents extérieurs agressifs, acides ou bases, agents oxydants, solvants ou rayonnements ultra-violets, est souvent moins bonne que celle du matériau « protégé » ;
- une contrainte majeure est l'adhésion du film au support. En effet, si celle ci est insuffisante, la couche va se détacher de son support, laissant éventuellement place à des phénomènes d'altération et d'irrégularité de surface.

Le recouvrement de matériaux industriels de type métallique ou minéral peut également être envisagé par des couches minces greffées par des fonctions thiols, monophosphonates ou siloxanes. Ces couches sont intéressantes dans la mesure où elles sont auto-assemblées sur les surfaces et qu'elles s'y lient par des liaisons covalentes. Cependant, certaines contraintes rendent l'utilisation de ces recouvrements de surface assez marginale.

Les thiols sont exclusivement réservés à la fonctionnalisation de surface à base d'or et présentent peu d'applications industrielles.

Les monophosphonates et les siloxanes permettent des greffages sur des surfaces oxydées avec des stabilités variables :
- Les surfaces de type titane ou silicium peuvent être recouvertes par une phase de trempage délicate à mettre en oeuvre dans des solutions anhydres de monophosphonates (EP1636023A1) en évitant toute force d'arrachement de surface synonyme de détérioration de la couche déposée. Cette phase est suivie d'une déshydratation à haute température qui peut être rédhibitoire pour certains types d'applications (molécules biologiques thermosensibles) ;
- Les siloxanes sont sensibles à l'hydrolyse. Aussi, une couche mince greffée de façon covalente peut rapidement se détériorer en fonction du pH et de la force ionique d'une solution aqueuse. Ceci rend également délicate leur liaison à la surface dans un milieu aqueux.

Le document FR-A-2 848 850 décrit un procédé de revêtement d'une surface minérale ou métallique (particules de fer) préalablement oxydés avec des composés gem-bisphosponiques, suivie de l'élimination (par lavage) de la composition de recouvrement et d'une déshydratation par filtration.

La présente invention concerne un nouveau procédé de recouvrement ou de fonctionnalisation de surfaces qui permet de pallier les inconvénients de procédés à base de thiols, monophosphonates ou siloxanes décrits précédemment, et qui présente les avantages suivants:
- le greffage d'une couche moléculaire en phase aqueuse ou dans un solvant organique non anhydre ;
- la non dégradation de molécules thermosensibles ;
- la simplicité de mise en oeuvre ;
- la réduction du coût de greffage ;
- le recouvrement ou la fonctionnalisation de surfaces métalliques ou minérales et plus généralement toute surface hydroxylée ou pouvant l'être, par des molécules organiques de type gem-bisphosphonique en couches minces auto-assemblées greffées sur la surface de façon permanente, par une liaison de type covalente.

La mise en oeuvre simple du procédé objet de la présente invention permet la réduction des coûts des procédés existants par l'emploi de matériels et matières premières simples. Cette procédure s'applique pour tous les types de BP et permet d'envisager de nombreuses applications industrielles, biologiques ou biomédicales.

La présente invention a ainsi pour premier objet un procédé de recouvrement d'un substrat métallique ou minéral par une couche moléculaire de fonctionnalisation, caractérisé en ce qu'il comprend les étapes successives suivantes :
a) oxydation préalable de la surface du substrat si celui-ci n'est pas déjà au moins partiellement hydroxylé de façon à disposer de fonctions hydroxyles à la surface du substrat,
b) mise en contact de la surface du substrat avec une composition liquide, gazeuse ou supercritique de recouvrement contenant des composés gem-bisphosphoniques, et/ou leurs sels toxicologiquement acceptables, jusqu'à auto-assemblage desdits composés gem-bisphosphoniques en une couche recouvrant ladite surface,
c) élimination de ladite composition liquide, gazeuse ou supercritique de recouvrement,
d) déshydratation thermique de la surface ainsi recouverte.

Afin d'augmenter le rendement de fixation des composés gem-bisphosphoniques sur les surfaces du substrat, on peut répéter au moins une fois la séquence a) à d) après l'étape d).

Dans le cadre de la présente invention, on entend par couche moléculaire de fonctionnalisation, une couche composée de molécules qui sont chacune ancrées au substrat par l'une au moins de leurs terminaisons et disposées les unes à côtés des autres. Les molécules sont ancrées au substrat de préférence par leurs terminaisons gem-bisphosphoniques et ne sont pas liées entre elles. Leur organisation en surface et les différents groupements chimiques qu'elles présentent permettent de modifier les propriétés chimiques, physiques ou biologiques des surfaces ainsi recouvertes. On peut de cette façon, rendre hydrophobe ou hydrophile une surface, ou greffer des groupements biologiquement actifs sur la surface. L'épaisseur de la couche moléculaire obtenue selon le procédé objet de la présente invention est avantageusement de l'ordre du nanomètre, c'est-à-dire comprise enture 0,1 nm et 50 nm.

On entend par "substrat hydroxylé", un substrat dont la surface présente des fonctions -OH sous la forme X-OH (X étant un élément constitutif de la surface). Plus la surface du substrat présente de fonctions -OH et plus le nombre de composés gem-bisphosphoniques fixés sur cette surface sera important.

Dans la pratique, on effectuera l'oxydation préalable de la surface du substrat de façon à disposer de suffisamment de fonctions hydroxyles sur la surface du substrat pour permettre la fixation des composés bisphosphoniques, lorsque ledit substrat n'en est pratiquement pas pourvu ou très peu. On peut aussi le faire lorsqu'on souhaite augmenter le nombre de fonctions hydroxyles déjà présentes, afin d'obtenir un recouvrement plus important de la surface par les composés bisphosphoniques.

Les sels toxicologiquement acceptables sont de préférence choisis parmi les sels de sodium, potassium, magnésium, calcium, ammonium.

Dans le cadre de la présente invention, des surfaces oxydées sont recouvertes par des monocouches autoassemblées de composés gem-bisphosphoniques car ces molécules présentent des propriétés intéressantes pour répondre aux contraintes posées par les thiols, les monophosphonates et les siloxanes. L'affinité des BP pour les surfaces oxydées est augmentée du fait de la multiplicité des groupements P - O. Cette propriété permet :
- un recouvrement des surfaces moins sensible aux forces d'arrachement. Le trempage peut être réalisé dans des bains puis les supports sont extraits et rincés avant d'être déshydratés ;
- un dépôt peut s'effectuer en solution aqueuse, contrairement aux monophosphonates et aux siloxanes où les molécules d'eau qui écrantent la surface rendent la liaison métal-molécule instable.
- l'obtention de surfaces recouvertes par une couche moléculaire de fonctionnalisation constituée de BP dont la liaison au substrat est de type covalente, rendant ainsi cette monocouche de BP stable à différentes contraintes mécaniques et chimiques à l'issue de la phase de déshydratation.

L'affinité des BP pour les surfaces métalliques ou minérales ainsi que pour différents oxydes ou alliages est limitée. Le simple contact d'une solution de BP avec la surface est suffisant pour établir une interaction électrostatique qui peut être durable dans le cas de surfaces d'acier inoxydable ou d'hydroxyapatite utilisé pour des applications biologiques (Portet et al., J of Colloid and Interface Science ; (2001) 238, 37-42) mais qui est beaucoup moins stable pour d'autres applications dans le cas par exemple du titane (Yoshinari et al., Biomaterials, (2001) 22, 709-715), du silicium ou du verre.

Le procédé objet de la présente invention permet le greffage de type covalent des BP sur des surfaces oxydées en utilisant des techniques de déshydratation par chauffage sous pression réduite qui permet de transformer cette interaction électrostatique en une liaison de type covalente P-O-X (X étant un élément constitutif de la surface).

Le procédé objet de la présente invention permet de recouvrir des substrats dont la surface libre comprend ou est constituée de titane, vanadium, chrome, manganèse, fer, cobalt, nickel, cuivre, zinc, aluminium, tungstène, zirconium, acier, acier inoxydable, ou leurs alliages ou oxydes, comme le saphir ou le rubis, de silicium ou germanium, éventuellement dopés, ou de leur oxydes, ou de quartz, de mica, de verre ou de calcaire.

Selon un mode de réalisation préféré, la surface libre du substrat est constituée de titane ou de ses alliages ou oxydes.

Selon un autre mode de réalisation préféré, la surface libre du substrat est constituée de silicium ou germanium, ou leurs alliages ou oxydes, éventuellement dopés, de quartz, de verre ou de calcaire.

Selon un autre mode de réalisation préféré, la surface libre du substrat est constituée d'aluminium, ou de ses alliages ou oxydes, tels que le saphir ou rubis.

Selon un autre mode de réalisation préféré, la surface libre du substrat est constituée de vanadium, chrome, manganèse, fer, cobalt, nickel, cuivre, zinc, tungstène, zirconium, acier ou acier inoxydable, ou leurs alliages ou oxydes.

Le procédé objet de la présente invention se caractérise en outre par le fait qu'il ne nécessite pas d'être mis en oeuvre dans des conditions anhydres.

Selon le procédé objet de la présente invention, la surface du substrat est mise en contact avec une composition liquide de recouvrement contenant des composés gem-bisphosphoniques jusqu'à auto-assemblage desdits composés gem-bisphosphoniques en une couche recouvrant ladite surface.

La composition de recouvrement peut être liquide, gazeuse ou supercritique.

Lorsqu'elle est liquide, la composition de recouvrement contenant les composés gem-bisphosphoniques peut être une composition aqueuse ou organique. Le solvant de la composition liquide est choisi de manière à permettre la solubilisation de ces composés gem-bisphosphoniques. Le solvant organique pourra être choisi parmi des solvants alcooliques, en particulier des alcools en C1 à C6, tels que l'isopropanol, l'éthanol, le méthanol, des aldéhydes, des cétones tels que l'acétone, des éthers tels que le diéthyléther ou le tétrahydrofurane ou des alcanes, notamment des alcanes en C1 à C8, ainsi que leurs mélanges.

La composition peut être gazeuse, le composé gem-bisphosphonique peut être notamment à l'état de vapeur.

Par "composition supercritique", on entend une composition qui se trouve dans un état de fluide supercritique. Le solvant de la composition supercritique est de préférence du dioxyde de carbone (CO₂).

La composition de recouvrement se présente avantageusement sous forme d'une solution, d'une suspension, d'une émulsion, d'un fluide supercritique, d'un aérosol ou d'une mousse. La teneur en composés gem-bisphosphoniques dans la composition liquide de recouvrement est avantageusement comprise entre 0,0001 et 20 % en poids, de préférence entre 0,001 et 5 % en poids.

La mise en contact de la composition liquides de recouvrement avec la surface du substrat est réalisée avantageusement par trempage, par spin-coating, par essuyage, par vaporisation, par aérosol ou par pulvérisation.

Dans la pratique, la mise en contact de la composition liquide de recouvrement avec la surface du substrat peut être réalisée par trempage du substrat dans une solution aqueuse contenant entre 0,001 et 5 % de composés gem-bisphosphoniques, pendant un temps compris entre 5 minutes et 3 heures, à température ambiante, avec ou sans agitation.

Lorsque la composition de recouvrement est gazeuse ou supercritique, la mise en contact avec la surface du substrat peut être réalisée à l'aide d'un réacteur dont la pression et la température sont contrôlables et qui permet l'injection d'un gaz tel que le CO2.

Après l'étape de mise en contact du substrat avec la composition de recouvrement, on procède à l'élimination de la composition de recouvrement, de façon à éliminer de la surface du substrat le solvant et tout le soluté gem-bisphosphonique qui ne s'est pas fixé au substrat lors de la mise en contact.

L'élimination de la composition de recouvrement peut être réalisée par rinçage, ou mécaniquement par égouttage, centrifugation ou évaporation.

Le substrat peut être en outre rincé notamment par immersion dans un solvant approprié, afin d'assurer une élimination complète du soluté non fixé.

Une fois que le dépôt de la couche moléculaire de composés gem-bisphosphoniques est réalisé, on procède à la déshydratation de la surface recouverte. Sans vouloir être lié à cette théorie, on suppose que la déshydratation de la couche gem-bisphosphonique formée sur le substrat permet de former des liaisons de type covalent entre les molécules gem-bisphosphoniques et les fonctions hydroxyles libres de la surface du substrat. L'étape de déshydratation de la surface est réalisée thermiquement, avantageusement sous pression réduite, notamment au moyen d'un lyophilisateur.

Dans la pratique, la déshydratation de la surface du substrat peut être réalisée par chauffage de celle-ci à une température comprise entre 20°C et 150°C, de préférence à environ 50°C, sous une pression comprise entre 0,01 mBar et 1 Bar, de préférence à 0,3 mbar, pendant un temps compris entre 1 et 72 heures, de préférence pendant environ 15 heures.

Toutes ces différentes étapes successives peuvent être répétées plusieurs fois de façon cyclique.

Les composés gem-bisphosphoniques contenus dans la composition liquide de recouvrement, permettant la mise en oeuvre du procédé objet de la présente invention, peuvent répondre formule (I) suivante : 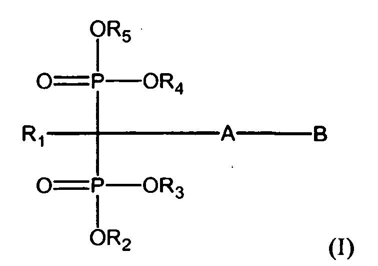 dans laquelle :
- R2, R3, R4, R5 représentent indépendamment l'un de l'autre un atome d'hydrogène ou un groupe alkyle en C1-C10, tel que méthyle ou éthyle ; de préférence R2, R3, R4, R5 sont identiques et représentent un atome d'hydrogène ;
- A représente une liaison chimique simple, -O-, -S- ou -NH-, de préférence une liaison chimique simple ;
- B représente:
   a) un groupement alkyle en C1-C100, linéaire ou ramifié, saturé ou non, la chaîne alkyle pouvant être substituée ou interrompue par 1 à 10 groupements aryle ; de préférence un groupement alkyle en C9-C20 ; ou
   b) un groupement -(CH₂)ₘ-X dans lequel m est un entier compris entre 1 et 100 et X est un groupement alkyle en C1-C100 saturé ou non, pouvant être perfluoré ou partiellement fluoré, la chaîne alkyle pouvant être substituée ou interrompue par 1 à 10 groupements aryle pouvant être perfluorés ou non;
   de préférence B représente un groupement alkyle en C9-C20 perfluoré ou partiellement fluoré; et
- R1 représente un atome d'hydrogène ou un groupement -OH, -NH₂, alkyle en C1-C100, pouvant être perfluoré ou partiellement fluoré ; R1 représente de préférence un groupement -OH ;
   ainsi que leurs sels toxicologiquement acceptables,
ou bien à la formule (I') suivante : 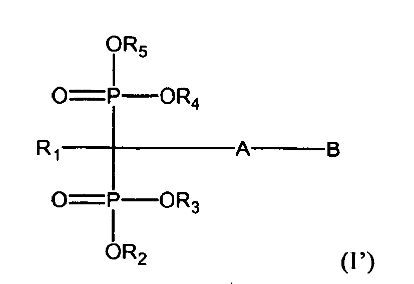 dans laquelle :
- R2, R3, R4, R5 représentent indépendamment l'un de l'autre un atome d'hydrogène ou un groupe alkyle en C1-C10, tel que méthyle ou éthyle ; de préférence R2, R3, R4, R5 sont identiques et représentent un atome d'hydrogène ;
- B représente un groupement -(CH₂)ₘ-D-E dans lequel :
   - m est un entier compris entre 1 et 100,
   - D est une liaison chimique simple ou un groupement -O-, -S-, -OCO-,-COO-, -COS-, -SCO-, -SCS-, -CONH-, -HNCO-, -HNCO-CF(CF₃)- ou -NH-CO-NH-NH-CS-NH-, et
   - E est un groupement: -(O-CF₂-CF₂)ₙ-OR_{f}, -(O-CF₂-CF(CF₃))ₙ-OR_{f}, - (O-CF(CF₃)-CF₂)ₙ-OR_{f} ou -(O-CF₂-CF₂-CF₂)ₙ-OR_{f}, où n est un entier compris entre 1 et 100 et R_{f} est un groupement alkyle perfluoré en C1-C10, ou bien un groupements 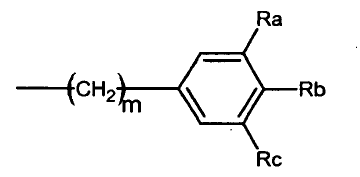 dans lequel, Ra, Rb, Rc représentent indépendamment l'un de l'autre un atome d'hydrogène, un alkyle en C1-C100 perfluoré ou un groupement: -(O-CF₂-CF₂)ₙ-OR_{f}, -(O-CF₂-CF(CF₃))ₙ-OR_{f}, -(O-CF(CF₃)-CF₂)ₙ-OR_{f} ou -(O-CF₂-CF₂-CF₂)ₙ-OR_{f}, où n et R_{f} sont tels que définis précédemment et m est un entier compris entre 0 et 20,
   - A représente :
      a) une liaison chimique simple, ou
      b) une fonction -O-, -S- ou -NH-, et
   - R1 représente un atome d'hydrogène ou un groupement -OH, -NH₂, alkyle en C1-C100, pouvant être perfluoré ou partiellement fluoré ; R1 représente de préférence un groupement -OH ;
ainsi que leurs sels toxicologiquement acceptables.

La présente invention a en particulier pour objet les composés gem-bisphosphoniques de formule (Ia) suivante : 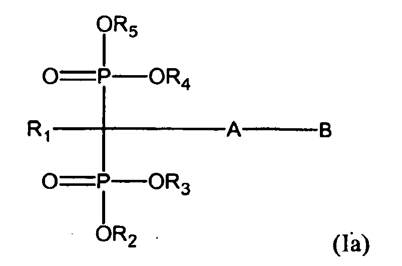 dans laquelle :
- R2, R3, R4, R5 représentent indépendamment l'un de l'autre un atome d'hydrogène ou un groupe alkyle en C1-C10, tel que méthyle ou éthyle ; de préférence R2, R3, R4, R5 sont identiques et représentent un atome d'hydrogène ;
- A représente une liaison chimique simple, -O-, -S- ou -NH-, de préférence une liaison chimique simple;
- B représente un groupement -(CH₂)ₘ-X dans lequel m est un entier compris entre 1 et 100 et X est un groupement alkyle en C1-C100 saturé ou non, pouvant être perfluoré ou partiellement fluoré, la chaîne alkyle pouvant être substituée ou interrompue par 1 à 10 groupements aryle pouvant être perfluorés ou non;
   de préférence, m est compris entre 1 et 20 et X représente un groupement alkyle perfluoré ou partiellement fluoré en C9-C20 ; de manière encore plus préférée, B représente un groupement alkyle en C9-C20 perfluoré ou partiellement fluoré; et
- R1 représente un atome d'hydrogène ou un groupements -OH, -NH₂, alkyle en C1-C100, pouvant être complètement ou partiellement fluoré ; R1 représente de préférence un groupement -OH ;
ainsi que leurs sels toxicologiquement acceptables.

« Aryle » désigne un reste hydrocarboné aromatique, perfluoré ou non, monocyclique ou bicyclique et comprend notamment les groupements phényle, 1-naphtyle ou 2-naphtyle.

« Alkyle en Ci-Cj » désigne un alkyle comprenant i à j atomes de carbone.

En raison de leur caractère hydrophobe, les composés gem-bisphosphoniques de formule (I), (I'), ou (Ia) peuvent être utilisés comme :
- agents de surface modifiant les propriétés de mouillabilité, de lubrification et d'adhésion de surfaces métalliques et/ou minérales,
- agents permettant d'augmenter la fiabilité de micro et nano-systèmes en limitant les tensions interfaciales entre deux surfaces recouvertes, ou entre une surface recouverte et une surface non recouverte, ou entre une surface recouverte et le milieux gazeux,
- agents permettant de faciliter le glissement de pièces mécaniques et/ou à éviter leur collage, notamment pour la lubrification de pièces mécaniques, de microsystèmes électromécaniques ou nanosystèmes électromécaniques, ou pour la lubrification des pièces mécaniques destinées à l'horlogerie, l'industrie automobile, médicale ou aéronautique,
- agents permettant de limiter la mouillabilité de surfaces (limitant la formation de givre et l'étalement de liquides à la surface),
- agents anti-graffitis en limitant leur prise sur les supports traités et en facilitant leur nettoyage.
- agent permettant de maintenir en place un fluide lubrifiant (augmentation de l'effet épilame).

Les propriétés de mouillabilité de la surface du support choisi peuvent induire de larges variations de comportement lorsqu'un liquide vient à son contact. Dans le cas où l'énergie de surface est très élevée (le cas de la plupart des métaux et des matériaux de construction minéraux par exemple), la majorité des liquides va s'étaler et pénétrer dans les anfractuosités du support : le matériau va être « mouillé », pouvant conduire à une augmentation des dégradations chimiques. Ce phénomène explique aussi l'aspect taché que prend un matériau poreux comme certaines pierres au contact de projections d'huile. Bien que ce phénomène puisse être profitable (en particulier pour la protection des matériaux de constructions par des huiles siccatives, des vernis...), il est souhaitable de le limiter en particulier par utilisation de revêtements de très faible énergie superficielle comme des monocouches de composés aliphatiques perfluorés ou non. Les applications envisageables sont :
- la constitution de revêtements anti-graffitis ;
- la facilitation de l'entretien des surfaces d'acier inoxydable dans les bâtiments;
- la limitation de la formation de givre sur les surfaces métalliques comme certaines ailes d'avion (ce qui induit une perte de portance), les carburateurs en aviation (induisant une perte de puissance), pour faciliter les échanges thermiques dans les installations de réfrigération (le givre formé sur les échangeurs est un très mauvais conducteur thermique)...

Cet aspect de faible mouillabilité est particulièrement important avec les surfaces dites « super-hydrophobes » qui sont constituées superficiellement de micro-aspérités hydrophobes (obtenues soit par ajout de microparticules soit par micro-érosion de la surface initiale, souvent complétées par étalement d'une couche hydrophobe très fine).

L'utilisation de couches de faible énergie superficielle (comme des acides gras par exemple ou des composés fluorés et perfluorés) est également largement répandue dans le domaine de la lubrification de pièces mécaniques. Cette notion de lubrification recouvre en réalité de très nombreux phénomènes physiques, de type collage des surfaces (dues aux aspérités de surface mais également énergies superficielles des matériaux en jeu), glissement sur des couches superficielles, « surfing » sur le liquide plus ou moins visqueux (lubrification « hydrodynamique »). Dans le cadre de lubrification de pièces métalliques, il est souhaitable de disposer de couches très fortement fixées qui conduisent à des surfaces de faible énergie superficielle. Dans ce domaine de la lubrification, un cas particulier est celui des microsystèmes électromécaniques MEMS (Micro Electro Mechanical Systems) ou nanosystèmes électromécaniques NEMS (Nano Electro Mechanical Systems) souvent réalisés en composés semi-conducteurs (comme du silicium ou du germanium), pour lesquels les contraintes de tenue dans le temps, de faible taille des paliers et d'absence de contamination des surfaces avoisinantes sont particulièrement importantes.

Il est ainsi souhaitable de disposer d'agents capables de fortement se fixer sur ces surfaces et de modifier les propriétés de mouillabilité au sens large du terme, soit en permettant une lubrification efficace, soit en limitant les phénomènes de collage et d'étalement des liquides.

La présente invention vise à palier les inconvénients de l'art antérieur en proposant les composés gem-bisphosphoniques de formule (I), ainsi que les compositions les contenant, qui sont particulièrement efficaces et adaptées au contrôle des propriétés superficielles de surfaces métalliques ou minérales et qui permettent une fonctionnalisation efficace contre le collage des surfaces, selon différents modes d'application possibles, notamment solutions aqueuses, alcooliques ou organiques, suspensions, mousses, poudres, aérosols, huiles.

La présente invention a donc pour objet :
- les composés gem-bisphosphoniques de formule (Ia) tels que définis ci-dessus ;
- le substrat fonctionnalisé susceptible d'être obtenu par le procédé tel que défini précédemment à partir des composés gem-bisphosphoniques de formule (I), (I') ou (Ia) tel que définis ci-dessus. Ce substrat peut être avantageusement du verre, un métal ou un de ses alliages tel qu'un acier, du titane, un oxyde de titane, un semi-conducteur, un microsystème électromécanique ou un nanosystème électromécanique ;
- l'utilisation de ce substrat fonctionnalisé dans des pièces mécaniques, microsystèmes électromécaniques ou nanosystèmes électromécaniques pour limiter les forces de collage ou faciliter le glissement entre deux pièces mécaniques ; ou dans des pièces mécaniques destinées à l'horlogerie, l'industrie automobile ou aéronautique.;
- l'utilisation des composés gem-bisphosphoniques de formule (I), (I') ou (Ia) tel que définis ci-dessus, comme agents de surface permettant de modifier les propriétés de mouillabilité, de lubrification et d'adhésion de surfaces métalliques ou minérales, en particulier pour la lubrification ou l'anti-collage de pièces mécaniques, de microsystèmes électromécaniques ou nanosystèmes électromécaniques, pour la lubrification des pièces mécaniques destinées à l'horlogerie ou pour augmente l'effet épilame, l'industrie automobile ou aéronautique, pour limiter la formation de givre ou l'étalement de liquide sur ces surfaces, ou comme agent anti-graffiti.

La présente invention a également pour objet des compositions utilisant une quantité efficace des composés gem-bisphosphoniques de formule (I), (I') ou (Ia) ou de leurs sels toxicologiquement acceptables, capables de se fixer durablement sur les surfaces minérales à protéger et aptes à diminuer le mouillage par des liquides aqueux et non aqueux, à limiter le collage ou/et à faciliter la lubrification des pièces mécaniques.

Les composés gem-bisphosphoniques de formule (I), (I') ou (la) sont plus spécialement destinés à modifier les propriétés de mouillabilité des surfaces métalliques et minérales à leur contact.

Selon un autre aspect, la présente invention concerne des compositions liquides comprenant au moins un composé gem-bisphosphonique de formule (I), (I') ou (Ia) tels que définis précédemment.

Selon un mode de réalisation, le composé gem-bisphosphonique de formule (I), (I') ou (Ia) est intégré dans une composition pour dépôt superficiel comprenant typiquement entre 0,0001 et 20 %, avantageusement entre 0,001 et 5 %, en poids du composé gem-bisphosphonique de formule (I), (I') ou (Ia). Par l'expression "composition pour dépôt superficiel", on entend une composition pour une application locale sur la surface à protéger telle qu'une composition liquide (par exemple solution ou suspension aqueuse, alcoolique, hydro-alcoolique, organique, de type émulsion, dans des fluides supercritiques...), un aérosol ou une mousse.

La composition peut être appliquée sur les surfaces à traiter par diverses techniques appropriées notamment la pulvérisation ou le trempage. D'autres moyens d'application possibles sont connus de l'homme du métier.

Exemple de composition de liquide utilisé par trempage, rinçage, dépôt par chiffon humide ou par aspersion:
- composé gem-bisphosphonique de formule (I), (I') ou (Ia) : 0,001 à 5%
- acétone : 95 à 99,999 %

Dans cette composition, la présence d'acétone facilite le mouillage de la surface et l'homogénéité du revêtement. De plus, l'évaporation de l'acétone permet d'obtenir à la surface de très hautes concentrations facilitant une adsorption rapide. L'acétone peut être avantageusement remplacée par des composés volatils (alcools de C1 à C6 en particulier alcool isopropylique, aldéhydes, cétones, éthers, alcanes de C4 à C8 en particulier).

Une telle composition ne laissant que peu de résidus solides après évaporation des liquides est particulièrement indiquée lorsque la surface doit être directement utilisée après application, en particulier pour les appareils et instruments devant être au contact de la nourriture ou de l'organisme humains

Selon un autre aspect, l'invention a pour objet l'utilisation d'un composé gem-bisphosphonique de formule (I), (I') ou (Ia) tel que décrit ci-dessus pour la fabrication d'une composition destinée à modifier les propriétés de mouillabilité du support. Une telle préparation peut également s'avérer performante pour produire des couches anti-collage et lubrifiantes.

Selon un autre aspect, la présente invention a pour objet un procédé pour contrôler le recouvrement de supports métalliques, par l'application d'une composition comprenant les composés gem-bisphosphoniques de formule (I), (I') ou (Ia) en quantité efficace, ou d'un sel toxico logiquement acceptable de ceux-ci.

Par le terme « quantité efficace », on entend que la quantité de composé gem-bisphosphonique appliquée permet, après recouvrement, de former un film monomoléculaire limitant significativement l'adhésion entre deux pièces recouvertes.

Le terme « toxicologiquement acceptable » signifie que les sels du composé ont les mêmes propriétés toxicologiques générales que la forme acide phosphonique libre et sont acceptables du point de vue de l'environnement. Parmi les sels possibles, on inclut notamment les sels de sodium ou de potassium, les sels de calcium ou de magnésium, ou des sels formés par des ligands organiques appropriés tels que des sels d'ammonium quaternaire.

Les composés gem-bisphosphoniques contenus dans la composition liquide de recouvrement, permettant la mise en oeuvre du procédé objet de la présente invention, peuvent également répondre à la formule (II) suivante : 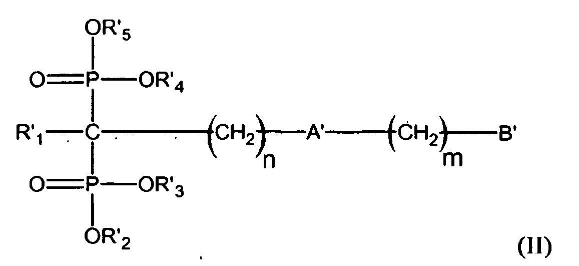 dans laquelle :
- n et m sont des entiers compris indépendamment l'un de l'autre entre 0 et 20; de préférence, n = 3, 4 ou 5 et m = 5 ;
- R'1 représente un atome d'hydrogène, un groupement -OH, une amine, ou un groupement alkyle en C1-C10, éventuellement fluoré; de préférence, R'1 représente un groupement -OH ;
- R'2, R'3, R'4, R'5 représentent indépendamment l'un de l'autre un atome d'hydrogène ou un groupement alkyle en C1-C10, tel que méthyle ou éthyle ; de préférence R'2, R'3, R'4, R'5 sont identiques et représentent un atome d'hydrogène ;
- A' représente un groupement L-Y où :
   L est une liaison chimique simple, un groupement -O-, -S-, -OOC-, -COO-, - CONH-, -HNCO-, -NH-CO-NH-, -NH-, -COS-, -SCO-, -SCS-, -NH-CS-NH- ; et
   Y est :
      a- une liaison chimique simple ;
      b- un groupement de formule 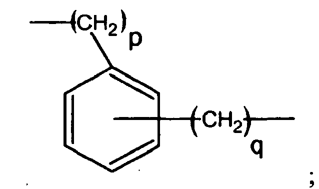 ou
      c- un groupement de formule 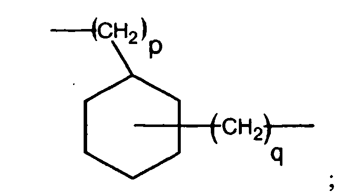 ou
      d- un groupement de formule 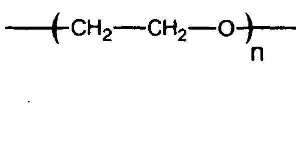 ou 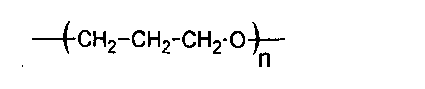 dans lesquels, n, p et q représentent indépendamment l'un de l'autre des entiers compris entre 0 et 100 ;
      de préférence A' représente un groupement -HNCO- ; et
   - B' représente :
      a) un groupement de formule 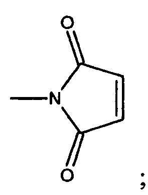 ou
      b) un groupement de formule 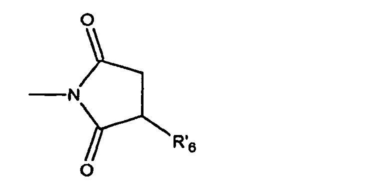
dans lequel R'6 représente un acide aminé, un peptide linéaire ou cyclique, notamment un peptide CRGD (cystéine - arginine - glycine - acide aspartique) ou RGDC, une protéine, une glyco-protéine, un acide nucléique simple ou double brin, un antibiotique, une hormone, un saccharide ou polysaccharide, une vitamine, un anticorps, un fragment d'anticorps, un haptène, un facteur de croissance, une cytokine, ou un radical pharmaceutiquement actif; de préférence, R'6 représente un acide aminé ou un peptide, tel que CRGD ou RGDC.

Dans la formule (II), A' représente avantageusement un groupe -HNCO- et n et m sont compris entre 1 et 10 indépendamment l'un de l'autre, et encore plus avantageusement n et m seront compris entre 3 et 6 indépendamment l'un de l'autre. R'6 représente préférentiellement un peptide, encore plus préférentiellement un peptide de type CRGD (cystéine - arginine - glycine - acide aspartique) ou RGDC. R'2, R'3, R'4 et R'5 sont de préférence identiques et représentent un atome d'hydrogène ou un groupement méthyle ou éthyle. R'1 représente de préférence un groupement -OH.

La présente invention a en particulier pour objet des composés gem-bisphosphoniques de formule (IIa) suivante : 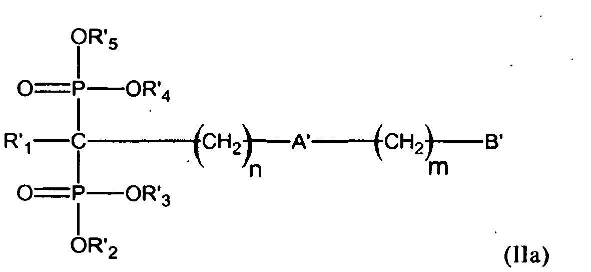 dans laquelle :
- n et m sont des entiers compris indépendamment l'un de l'autre entre 0 et 20; de préférence, n = 3, 4, ou 5 et m = 5 ;
- R'1 représente un atome d'hydrogène, un groupement -OH, une amine ramifiée ou non, ou un groupement alkyle en C1-C10, éventuellement fluoré; de préférence R'1 représente un groupement -OH ;
- R'2, R'3, R'4, R'5 représentent indépendamment l'un de l'autre un atome d'hydrogène ou un groupement alkyle en C1-C10, tel que méthyle ou éthyle ; de préférence R'2, R'3, R'4, R'5 sont identiques et représentent un atome d'hydrogène ;
- A' représente un groupement -L-Y- où :
   L est une liaison chimique simple, un groupement -O-, -S-, -OOC-, -COO-, - CONH-, -HNCO-, -NH-CO-NH-, -NH-, -COS-, -SCO-, -SCS-, -NH-CS-NH- ; et
   Y est :
      a- une liaison chimique simple;
      b- un groupement de formule 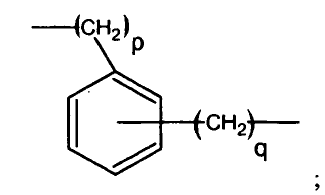 ou
      c- un groupement de formule 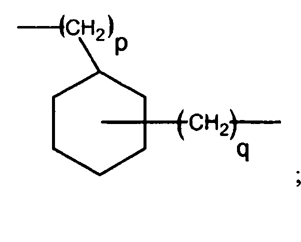 ;ou
      d- un groupement de formule 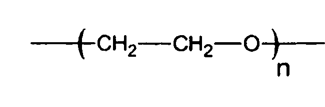 ou 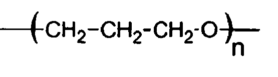
      dans lesquels n, p et q représentent indépendamment l'un de l'autre des entiers compris entre 0 et 100 ; de préférence compris entre 0 et 10 ;
      de préférence, A' représente un groupement -HNCO- ; et
   - B' représente un groupement de formule 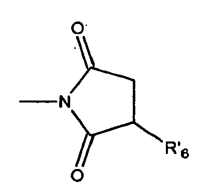
dans lequel R'6 représente un acide aminé, un peptide linéaire ou cyclique, notamment un peptide CRGD ou RGDC, une protéine, une glyco-protéine, un acide nucléique simple ou double brin, un antibiotique, une hormone, un saccharide ou polysaccharide, une vitamine, un anticorps, un fragment d'anticorps, un haptène, un facteur de croissance, une cytokine, ou un radical pharmaceutiquement actif, de préférence R'6 représente un peptide CRGD ou RGDC;
ainsi que leurs sels toxico logiquement acceptables.

Les composés gem-bisphosphoniques de formule (II) ou (IIa), ainsi que leurs précurseurs, peuvent être utilisés comme agents de surface modifiant les propriétés d'adhésion d'entités biologiques (molécules, cellules, tissus). Les applications visées sont notamment :
- de fonctionnaliser des capteurs (biochips, biosensors) de façon à accroître leur spécificité vis-à-vis de molécules cibles ;
- de fonctionnaliser des surfaces implantables (greffes, implants dentaires, prothèses orthopédiques) de façon à favoriser la colonisation cellulaire ;
- de fonctionnaliser des billes ou d'autres surfaces oxydées utilisées pour le diagnostic ou comme médicament.

Dans le domaine de la fonctionnalisation de surface par des molécules d'intérêt biologique, il existe de nombreuses techniques de greffage covalent de molécules comme décrit ci-dessus. Cependant ces techniques s'accompagnent systématiquement d'inconvénients majeurs quant à une exploitation industrielle de celles-ci.

Dans de nombreuses applications, des silanes ou siloxanes sont utilisés pour fonctionnaliser des surfaces dans le but d'y fixer des molécules présentant une affinité particulière pour des composés biologiques (peptides, protéines, acides nucléiques, anticorps...). La liaison de molécules comme par exemple les triméthoxysilanes se fait par réaction avec des surfaces oxydées par formation d'une liaison Si-O-Métal mais également par une réticulation entre les différentes molécules monomériques (liaisons Si-O-Si) ce qui assure une certaine stabilité à la couche formée. Ces structures bi ou tridimensionnelles sont néanmoins peu stables car les liaisons Si-O-Métal et Si-O-Si sont hydrolysables dans certaines conditions de pH ce qui rend leur utilisation dans des milieux biologiques complexe sur de longues durées ou de façon régénérable.

Dans d'autres applications, des acides gem-bisphosphoniques ayant une activité biologique sont utilisés pour le recouvrement d'objets particulaires à base d'oxyde de fer (brevet W02004058275). Les molécules décrites sont synthétisées une fois la fonction gem-bisphosphonique greffée sur le support, ceci aboutit à des rendements et des puretés de fonctionnalisation non contrôlés ce qui rend la technologie complexe à mettre en oeuvre sur le plan industriel.

Le procédé objet de la présente invention permet de disposer soit de molécules de recouvrement actives immédiatement après greffage, soit de précurseurs polyvalents à ces molécules.

La synthèse d'une molécule porteuse d'une fonction activée de type maléimidyle a été réalisée en une étape grâce à un mode opératoire décrit dans le brevet US5409911. Ce précurseur permet ensuite de greffer de façon univoque et rapide, tout type de molécule porteuse d'une fonction thiol.

Cette famille de molécules a permis de synthétiser plusieurs nouveaux acides gem-bisphosphoniques porteurs de peptides et notamment le peptide CRGD (arginine - glycine - acide aspartique - cystéine) ou RGDC spécifique de l'adhésion de cellules comme par exemple les ostéoblastes.

Ces molécules d'intérêt biologique tout comme leurs précurseurs, utilisés selon le premier principe de cette invention, permettent la fonctionnalisation de surfaces métalliques ou minérales de façon durable. Il est alors possible de disposer de surfaces soit hautement spécifiques lorsque la fonction d'intérêt est greffée, soit hautement polyvalentes lorsque les molécules précurseurs sont disponibles sur la surface pour une fonctionnalisation ultérieure.

La présente invention a donc pour objet :
- les composés gem-bisphosphoniques de formule (IIa) tels que définis ci-dessus ;
- le substrat fonctionnalisé susceptible d'être obtenu par le procédé tel que défini précédemment à partir des composés gem-bisphosphoniques de formule (II) ou (IIa) tel que définis ci-dessus ;
- l'utilisation des composés gem-bisphosphoniques de formule (II) ou (IIa) tels que définis ci-dessus, ou du substrat fonctionnalisé susceptible d'être obtenu selon le procédé tel que défini précédemment, comme agents de fonctionnalisation de surfaces métalliques ou minérales, notamment pour le greffage de molécules biologiquement actives sur des surfaces métalliques ou minérales, notamment pour augmenter l'adhésion d'ostéoblastes sur des prothèses orthopédiques ou des implants dentaires, favoriser la minéralisation ou modifier l'équilibre des activités ostéoblastiques et ostéoclastiques, ou pour fonctionnaliser des surfaces de silice pour la purification, l'adhésion ou la reconnaissance d'entités biologiques.

La présente invention a également pour objet des compositions utilisant une quantité efficace des composés gem-bisphosphoniques de formule (II) ou (IIa)..

Selon un mode de réalisation, le composé gem-bisphosphonique de formule (II) ou (IIa) est intégré dans une composition pour dépôt superficiel de concentration molaire comprise entre 10⁻¹ et 10⁻¹⁵ mol/L du composé gem-bisphosphonique de formule (II) ou (IIa). Par les termes composition pour dépôt superficiel, on entend une composition pour une application locale sur la surface à protéger telle qu'une composition liquide (par exemple solution ou suspension aqueuse, alcoolique, hydro-alcoolique, organique, de type émulsion, dans des fluides supercritiques...), un aérosol ou une mousse.

Les composés gem-bisphosphoniques contenus dans la composition liquide de recouvrement, permettant la mise en oeuvre du procédé objet de la présente invention, peuvent également répondre à la formule (III) suivante : 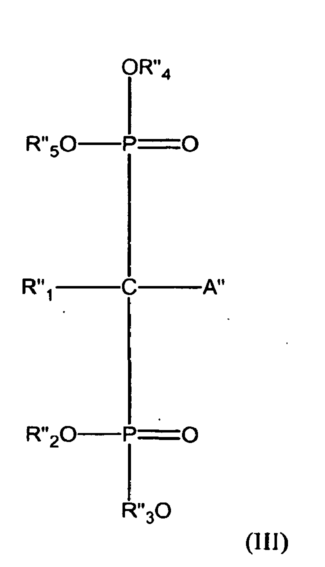 dans laquelle :
- R"1 représente un atome d'hydrogène, un groupe -OH, -NH2, un groupe alkyle en C1-C10, éventuellement fluoré ;
- R"2, R"3, R"4, R"5 représentent indépendamment l'un de l'autre un atome d'hydrogène ou un groupe alkyle en C1-C10, tel que méthyle ou éthyle ; de préférence R"2, R"3, R"4, R"5 sont identiques et représentent un atome d'hydrogène ; et
- A" représente :
   a. un groupement de formule

      -CH₂O-(CH₂CH₂O)ₙ-X

      dans lequel n est un entier compris entre 0 est 100 et X représente un hydrogène ou un groupement alkyle en C1-C5, de préférence X représente un groupement méthyle ; ou
   b. un groupement de formule 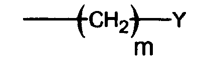 dans lequel m est un entier compris entre 0 et 20, de préférence compris entre 3 et 6, et Y représente un groupement -NH-CO-CH₃, -CO-NH-CH₃,-N(CH₃)-CO-CH₃ ou -CO-N(CH₃)₂.

Avantageusement, X représente un groupement méthyle, m est compris entre 1 et 10 et encore plus avantageusement m sera compris entre 3 et 6.

Les composés gem-bisphosphoniques de formule (III) tel que définis ci-dessus peuvent être utilisés comme molécules anti-biocontamination ou comme agent de biocompatibilité, notamment pour empêcher ou limiter la fixation de macromolécules, des microorganismes ou de biofilm, sur des surfaces métalliques ou minérales.

La présente invention a donc pour objet l'utilisation des composés gem-bisphosphoniques de formule (III) tels que définis précédemment, ou du substrat fonctionnalisé susceptible d'être obtenu selon le procédé tel que défini précédemment, contre la biocontamination de surfaces métalliques ou minérales ou comme agent de biocompatibilité, notamment pour empêcher ou limiter la fixation de macromolécules sur des surfaces solides telles que des surfaces métalliques ou minérales, pour empêcher ou limiter la fixation de microorganismes, de préférences de bactéries, sur des surfaces solides telles que des surfaces métalliques ou minérales, pour empêcher ou limiter la formation et le développement d'un biofilm sur les surfaces solides, notamment métalliques ou minérales.

Lesdites surfaces métalliques appartiennent notamment au groupe comprenant le fer, l'acier inoxydable, le chrome, l'aluminium, le zinc, le titane, le tungstène, le plomb ou le cuivre, ainsi que les oxydes, les alliages ou composites contenant au moins l'un de ces métaux et les surfaces minérales appartiennent au groupe comprenant le silicium et ses dérivés, les matériaux silicieux, les surfaces calciques, les surfaces céramiques ou dentaires.

Lesdites surfaces sont notamment la surface de matériel industriel, agroalimentaire ou hospitalier, de bâtiments, de constructions ou de véhicules terrestres, aériens ou maritimes, de matériel de climatisation ou de réfrigération ou bien la surface d'instruments chirurgicaux, de prothèses, d'instruments de dentisterie ou de capteurs biologiques et médicaux.

La présente invention a également pour objet des compositions utilisant une quantité efficace des composés gem-bisphosphoniques de formule (III), comme celles décrites pour les composés de formule (I).

La présente invention est illustrée par les exemples suivants.
La figure 1 représente les angles de contact avec l'eau, le glycérol et le diiodométhane de supports silicium traités ou non par différentes molécules (A, B, C et D).
La figure 2 représente la tension superficielle de supports silicium traités ou non par différentes molécules (A, B, C et D).
La figure 3 représente les angles de contact avec l'eau, le glycérol et le diiodométhane de supports titane traités ou non par différentes molécules (A, B et D).
La figure 4 représente la tension superficielle de supports titane traités ou non par différentes molécules (A, B et D).
La figure 5 représente les angles de contact avec l'eau, le glycérol et le diiodométhane de supports en Titane traités ou non par un BP porteur d'une fonction de fonctions réactives (molécule D, D + maléimide et D + maléimide + thiol).
La figure 6 représente les angles de contact avec l'eau, le glycérol et le diiodométhane de supports en verre traités ou non par la molécule A.
La figure 7 représente la tension superficielle de supports de verre traités ou non par la molécule A.
La figure 8 représente les angles de contact avec l'eau, le glycérol et le diiodométhane de supports en acier inoxydable traités ou non par différentes molécules (A et B).
La figure 9 représente la tension superficielle de supports en acier inoxydable traités ou non par différentes molécules (A et B).
La figure 10 représente la cinétique de formation de substance minérale par des cellules SaOS2 sur des supports en titane traités ou non par la molécule E.
La figure 11 représente le schéma de synthèse de la molécule B.

### I) Composés gem-bisphosphoniques selon l'invention

### Exemple 1 : Synthèse de l'acide 1-hydroxydodecyl 1-1 bisphosphonique (molécule C)

Des résultats particulièrement concluants ont été obtenus avec l'acide 1-hydroxydodecyl 1-1 bisphosphonique, synthétisée suivant la méthode de Lecouvey, et al. (Tetrahedron letters 42, 2001, 8475-8). 0,8 g de chlorure d'acide dodécylique (3,46 10⁻³ mol) est mélangé à 2,43 ml de tris(triméthylsilyl)phosphite (7,28 10⁻³ mol) sous un flux d'argon. Le mélange est agité pendant deux heures. Le produit formé est hydrolysé dans 40 ml de méthanol à température ambiante pendant une heure. Le solvant est évaporé et le produit séché au dessiccateur sous vide. Caractérisations : RMN ³¹P, D2O, δ :21,7 ppm.

### Exemple 2 : Préparation de l'acide 1-hydroxy-2H,2H-perfluorodécan-1,1- bisphosphonique (molécule A)

L'acide 2H,2H-perfluorodécanoique (4.6 g, 9.6 mmol) est chauffé en présence de 7 mL (10 éq.) de chlorure de thionyle à 80 °C sous argon pendant 17 h. Le chlorure de thionyle en excès est évaporé et le résidu est séché sous vide. Le solide obtenu est utilisé pour l'étape suivante sans purification complémentaire.

Après avoir refroidi sous argon le chlorure d'acyle (9.6 mmol) dans un bain eau-glace, on ajoute lentement 12.8 mL (4 éq.) de tris(trimethylsilyl)phosphite (P(OSiMe₃)₃). La suspension est réchauffée à température ambiante et après quelques minutes une solution limpide est obtenue. Le P(OSiMe₃)₃ en excès est évaporé sous pression réduite (60 °C) et le résidu est dissous dans 10 mL de méthanol anhydre puis agité à température ambiante sous argon pendant 2h. Après concentration du mélange, le solide obtenu est recristallisé dans l'acétone bouillant.

Masse obtenue : 1.6 g

Rendement : 25 %

### Exemple 3 : Préparation de l'acide 2H,2H,4H,4H,5H,5H,6H,6H-perfluoro-[7-aza-1-hydroxy-9,12,15,18-tetramethyl-10,13,16,19-tetraoxa-8-oxo]-docosylidene-1,1-bisphosphonique (molécule B)

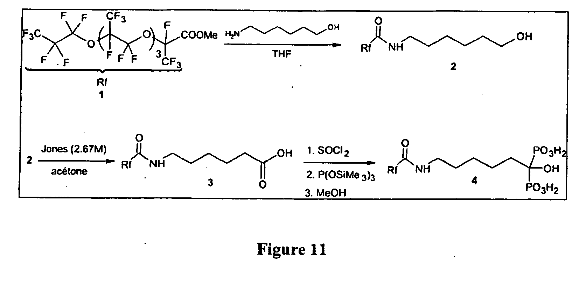

La molécule B peut être préparée en quatre étapes suivant le schéma de synthèse représenté dans la figure 11. Dans un premier temps, le 6-aminohexan-1-ol est acylé par l'ester méthylique PFPE **1** dans le THF à température ambiante pour conduire à l'amide **2** correspondant. La fonction alcool est ensuite oxydée en acide carboxylique **3** par action du réactif de Jones. Enfin le composé **3** est transformé en acide bisphosphonique **4** via un chlorure d'acide.

Le mode opératoire est décrit ci-après :
- Dans un monocol de 50 mL est dissous le 6-aminohexanol (1.25g ; 10.7 mmol) dans 15 mL de THF anhydre sous argon. L'ester méthylique **1** (3g ; 3.56 mmol) est ajouté en une fois. Le mélange biphasique devenu homogène et limpide après quelques minutes est agité à température ambiante pendant 17 heures. Après concentration au rotavapor, le sirop obtenu est repris dans l'AcOEt (25 mL) lavé avec une solution d'acide chlorhydrique 1N puis à l'eau. La phase organique est séchée (MgSO₄) filtrée puis concentrée sous vide (rotavapor puis pompe à palette). On obtient une huile incolore de la molécule **2.**
- L'alcool **2** (3.1 g, 3.3 mmol) est dissous dans 40 mL d'acétone. Une solution 2.67 M du réactif de Jones est ajoutée goutte à goutte. Après 15 minutes d'agitation à température ambiante, quelques gouttes d'isopropanol sont ajoutées puis le mélange est filtré, concentré, repris dans l'AcOEt et lavé deux fois à l'eau. La phase organique est séchée, filtrée puis concentrée sous vide (rotavapor puis pompe à palette). L'acide carboxylique **3** est obtenu sous forme d'une huile incolore.
- L'acide carboxylique **3** (3.1 g ; 3.3 mmol) est mélangé sous argon avec 8 mL de chlorure de thionyle. Le mélange est ensuite chauffé à reflux pendant 45 minutes, puis concentré sous vide.Le sirop obtenu est placé sous argon puis additionné de P(OSiMe₃)₃ (2.5 éq, 2.75 mL). La solution obtenue est agitée sous argon pendant 2h, concentrée puis additionnée de 10 mL de méthanol. Après 1h d'agitation le mélange est concentré. Le sirop obtenu est lavé à l'eau. La molécule B est ensuite séchée à la pompe à palette.

### Exemple 4 : Préparation de l'acide 1-hydroxy-3,6,9,12,15-pentaoxa-hexadecylidene-1,1-bisphosphonique (molécule F)

L'acide (3,6,9,12,15-pentaoxa)hexadécanoique (5.8g, 26.1 mmol), obtenu selon le protocole décrit par Lele et Al. (1998, Journal of Applied Polymer Science 70: 883-890) est mélangé avec 5 équivalents (10,7 g) d'acide phosphoreux puis chauffé à 65°C sous argon. Après 30minutes, on ajoute 6.8 mL de PCl₃ goutte à goutte puis le mélange est agité pendant 17h. Après addition de 25mL d'eau, le mélange réactionnel est chauffé à 100°C pendant 4 heures. Après concentration, le sirop est lavé à chaud à l'acétate d'éthyle, décanté puis séché sous pression réduite.

### Exemple 5 : Préparation d'un bisphosphonate-cystéine (molécule G)

Un équivalent de (R)-cystéine (6,2 mg) est additionné par sa fonction thiol à la double liaison conjuguée de l'acide 4-N-(6-maleimidohexanoyl) aminobutane-1-hydroxy -1,1-bisphosphonique synthétisé selon US 5409911 (25 mg; 0.051 mmol, 700 µL de solution aqueuse pH 7, D₂O). La réaction est contrôlée par RMN ¹H en suivant la disparition du signal des protons éthyléniques (δ=6.81 ppm) du maléimide.

### Exemple 6 : Préparation d'un acide gem-bisphosphonique porteur d'un peptide RGDC (molécule E)

5 mg de RGDC (Bachem - Suisse) sont dissout dans 1.45 mL d'eau osmosée ; le pH est ajusté à 7 par ajout de NaOH 0,1N (120µL). Cette solution est ajoutée à une solution de l'acide 4-N-(6-maleimidohexanoyl) aminobutane-1-hydroxy -1,1-bisphosphonique synthétisé selon US 5409911 (1 éq.). Le mélange est agité à température ambiante pendant une heure. Le solvant est ensuite évaporé sous pression réduite. Le produit désiré est obtenu sous forme d'une poudre blanche dans des rendements quantitatifs (pureté RMN³¹P >90%)

### Exemple 7 : Correspondance des molécules avec leur solvant

| Molécule | Formule Développée | solvant |
|---|---|---|
| Molécule A | 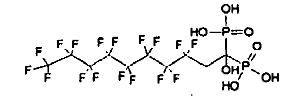 | acétone |
| Molécule B | 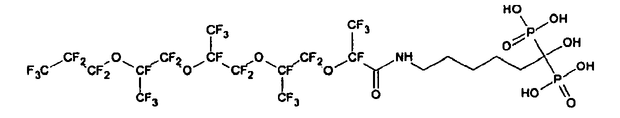 | acétone |
| Molécule C | 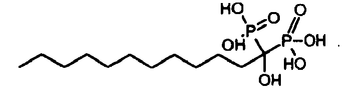 | acétone |
| Molécule D | 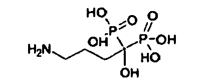 | H₂O |
| Molécule E | 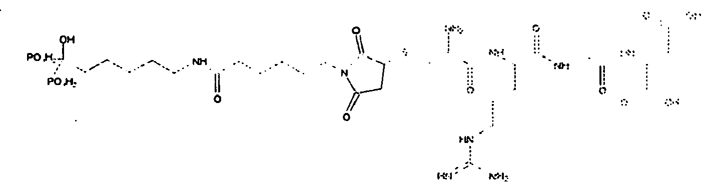 | H₂O |
| Molécule F | 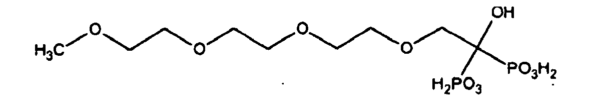 | H₂O |
| Molécule G | 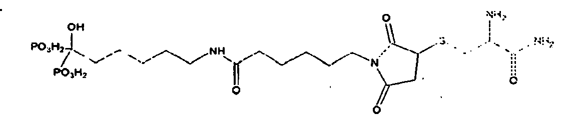 | H₂O |

### II) Recouvrement de surfaces avec les composés gem-bisphosphoniques selon l'invention,

### Exemple 8 : Mode de recouvrement de surface de silicium

Les surfaces sont oxydées avant traitement par les bisphosphonates comme décrit dans par Hanson et al. (2003, JACS, 125, 16074-80). Pour cela, les substrat de silicium (Si - 20mΩ.cm) subissent un traitement par une solution de H₂SO₄ 98% et H₂O₂ 35% (1:3, v:v) à reflux pendant 45 min, un rinçage à l'eau osmosée, puis un nouveau traitement par un mélange HCl 37% et H₂O₂ 35% (1:1 v:v) à 80°C pendant 15 minutes suivi d'un rinçage à l'eau osmosée.

Les supports de Si (1cm x 1cm) sont ensuite séchés puis sont plongés dans une solution de BP (10⁻³mol/L) à température ambiante sans agitation (molécules A, B, C et D décrites ci-dessus). Après trois heures, elles sont prélevées avec précaution à l'aide de pinces en prenant soin de ne pas griffer les surfaces puis rincées par immersion dans le solvant adapté (eau ou acétone).

La fixation par déshydratation du recouvrement a été effectuée par lyophilisation à une température de 50°C sous un pression de 0.3 mbar pendant 15 heures.

Pour chaque condition expérimentale, un échantillon référence a été réalisé sans molécule de recouvrement (Référence).

Tous ces supports subissent finalement un lavage aux ultrasons afin d'éliminer les potentielles multicouches ainsi que les molécules non fixées sur la surface.

Ces lavages sont réalisés comme suit :
- les supports sont plongés dans un large excès de solvant
- sonication 15 minutes, élimination du solvant (2 fois)
- séchage des supports à l'étuve

### Exemple 9 : Mesure de l'efficacité de fonctionnalisation par mesures des angles de contact et calcul des tensions superficielles de supports en Si

Les liquides tests sont l'eau ultra pure, le glycérol, le diiodométhane, à température ambiante.

L'estimation de la fixation des différentes molécules A, B, C, et D sur les supports est effectuée de façon qualitative par mesure des angles de contact entre les surfaces modifiées et différents liquides (en fonction des caractéristiques polaires et apolaires de ces derniers). Les solvants utilisés sont l'eau osmosée, le glycérol et diiodométhane. Ces mesures sont comparées avec celles réalisées sur un support non traité par ces produits (Référence).

La modification de la surface traitée par les BP se traduit par la modification des angles de contact envers ces différents liquides, ceci étant lié à la variation des caractéristiques polaires et apolaires de la tension superficielle. Les composantes de la tension superficielle liées à ces molécules sont calculées en utilisant l'équation de Fowkes. La significativité des résultats est calculée par un test t de Student.

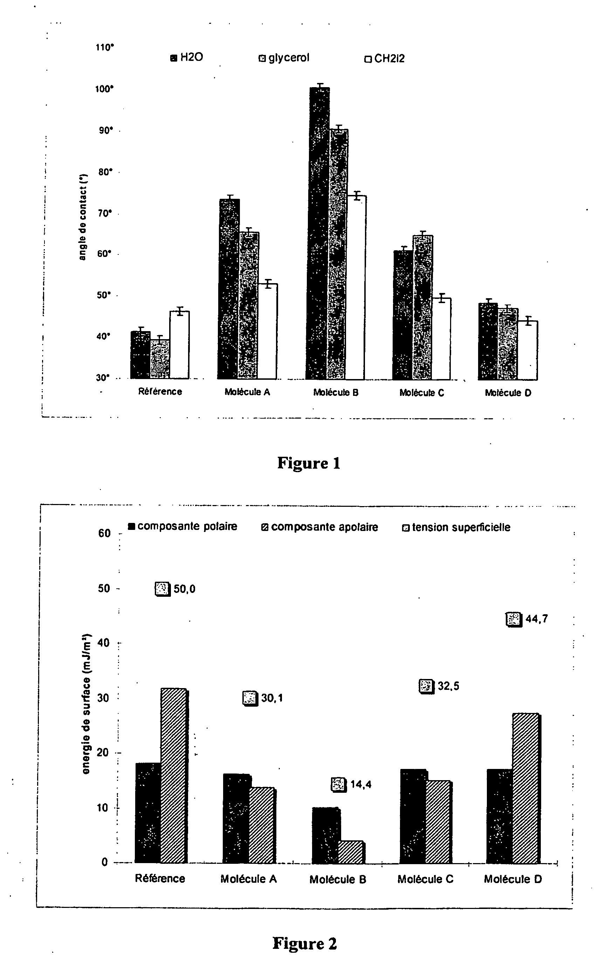

Ces résultats montrent que :
- les angles de contacts sont modifiés de façon significative (p<0.01) avec ces trois liquides vis à vis d'un support non traité quel que soit le BP ou le solvant utilisés (figure 1).
- les BP modifient les surfaces et que la déshydratation à 50°C sous pression réduite assure une fixation durable ;
- les énergies de surface, en particulier polaires, sont très largement diminuées, avec des valeurs homogènes quelques soient les supports de silicium (figure 2).

### Exemple 10 : Fonctionnalisation d'une surface Si déjà greffée

De façon à rendre hydrophobe une surface initialement rendue hydrophile grâce au greffage de la molécule D (exemple 8) sur un support en silicium, la surface a ensuite été mise en contact avec du chlorure d'acide hexadécanoique (10⁻² mol/L) dans le chlorure de méthylène pendant 2 heures. A l'issue de cette phase le support est rincé 2 fois par 3mL de CH₂Cl₂ puis a subit une phase de lavage par ultrasons comme décrite dans l'exemple 3.

Afin de montrer la disponibilité des fonctions réactives de molécules gem-bisphosphoniques greffées sur une surface en silicium, les angles de contact de gouttes d'eau posées sur ces substrats ont été mesurés avant et après réaction avec l'acide hexadécanoique.

Les résultats obtenus sont les suivants :

| | Avant réaction | Après Réaction |
|---|---|---|
| Angles de contact (°) | 48,7 +/- 2,3 | 85,3 +/- 6.6 |

La surface sur laquelle l'acide gras a réagi est devenue beaucoup plus hydrophobe qu'initialement où la molécule D apportait un caractère hydrophile marqué à la surface.

### Exemple 11 : Mode de recouvrement de surfaces de Titane

Des supports en titane commercialement purs de grade 2 (cpTi ; diamètre 8 mm épaisseur 3 mm) ont été mis en forme selon le protocole suivant : les disques ont été usinés par un appareil ASTM B 348-97 bars (Laboratoire Lemouel, Angers, France), polis avec de la poudre de carbure de silicium (calibre 320 -4000), ébarbés, et à nouveau polis. Les supports ont ensuite été lavés aux ultrasons dans un bain de CH₂Cl₂ (10min) puis dans un bain d'acétone (10 min) et enfin dans un bain de méthanol (5 min, 2 fois). A l'issue de cette phase, les échantillons subissent un traitement thermique à 200°C pendant 15 heures. (Danahy et al. Langmuir 2004, 20, 5033-37).

Les plaques sont entièrement immergées dans une solution de BP (10⁻³M) à température ambiante sans agitation. Après trois heures, elles sont prélevées avec précaution à l'aide de pinces en prenant soin de ne pas griffer les surfaces réservées au contact puis rincées par immersion dans le solvant correspondant (eau ou acétone). Le recouvrement a été effectué avec les molécules A , B et D (exemple 3) ainsi que la molécule E.

La fixation par déshydratation du recouvrement a été effectuée par lyophilisation à une température de 50°C sous un pression de 0.3 mbar pendant 15 heures. Pour chaque condition expérimentale, un échantillon référence a été réalisé sans molécule de recouvrement. Tous ces supports ont finalement subi un lavage aux ultrasons afin d'éliminer les potentielles multicouches ainsi que les molécules non fixées sur la surface.

Ces lavages on été réalisés comme suit :
- les supports sont plongés dans un large excès de solvant
- sonication 15 minutes, élimination du solvant (2 fois)
- séchage des supports à l'étuve

### Exemple 12 : Mesure de l'efficacité de fonctionnalisation par mesures des angles de contact et calcul des tensions superficielles de supports en Ti

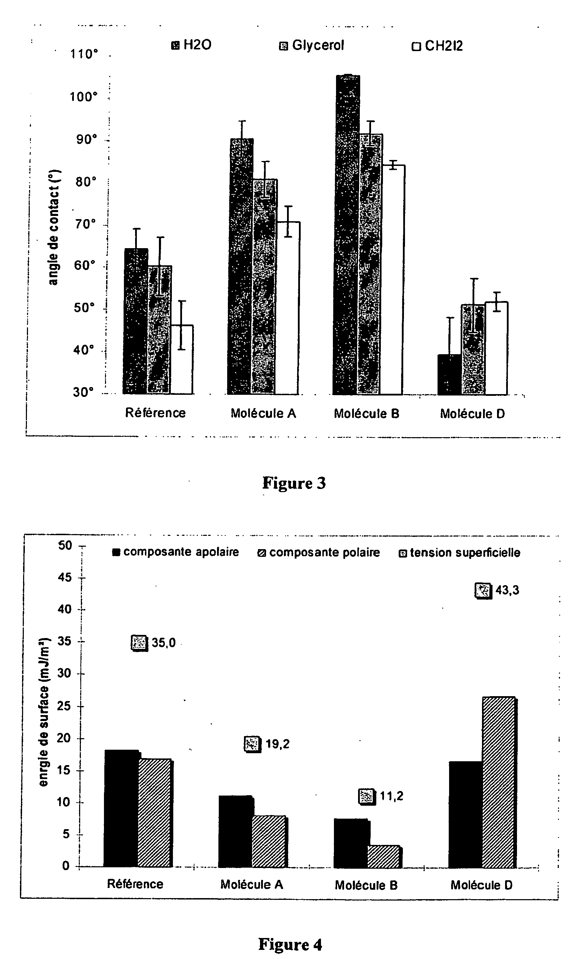

Le même protocole d'analyse de surface que dans l'exemple 10 est appliqué aux supports de titane recouverts des molécules A, B ou D, selon le procédé de décrit dans l'exemple 11.
- les angles de contacts et les tensions superficielles sont modifiés de façon significative (p<0.01) avec ces trois liquides vis à vis d'un support non traité quel que soit le BP ou le solvant utilisés (figure 3 et 4).
- les BP modifient les supports et que la déshydratation à 50°C et sous pression réduite assure une fixation durable ;
- il est possible de greffer des molécule de type BP sur du titane de façon durable et résistante à un nettoyage par ultrasons.
- Il est possible de greffer des bisphosphonates hydrophiles et des bisphosphonates hydrophobes sur du titane.

### Exemple 13 : Fonctionnalisation de supports en Ti par un BP porteur d'une fonction réactive (maléimidyle) permettant le greffage d'un groupement chimique

Le même protocole de recouvrement de surface que dans l'exemple 11 est appliqué. Celui-ci est réalisé avec les molécules D.

Dans un deuxième temps les supports ont été traités par une solution de maleimido hexanoate de succinimidyle (10⁻³M dans le THF) pendant 48 heures, puis rincée au THF. Ceci a permis d'obtenir des supports porteurs de l'acide 4-N-(6-maleimidohexanoyl) aminobutane-1-hydroxy -1,1-bisphosphonique. Ces derniers ont enfin été traités par une solution de dodécane thiol (10⁻³M dans l'acétone) pendant 15 heures puis rincées à l'acétone.

### Exemple 14 : Validation de la fonctionnalisation des surfaces de l'exemple 13.

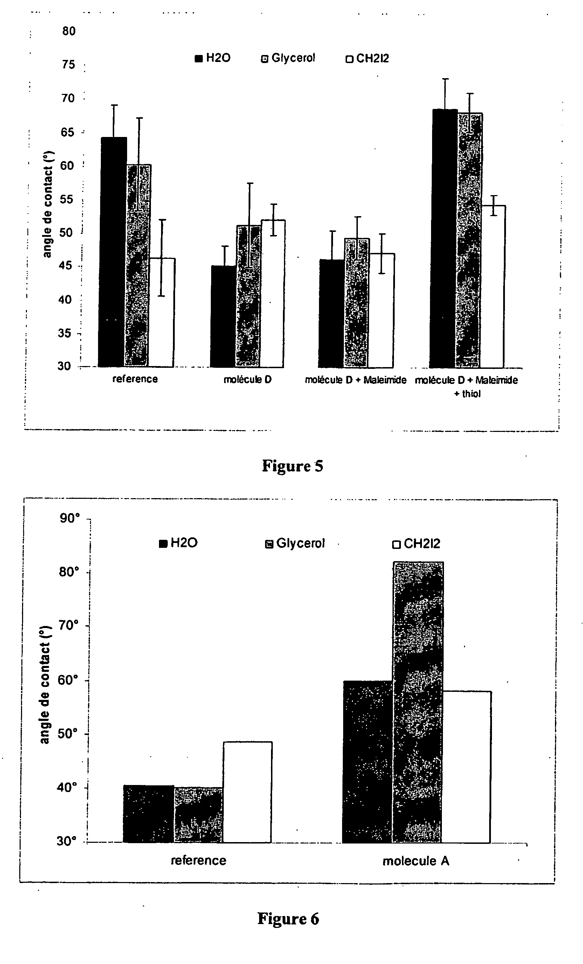

Pour valider la fonctionnalisation, une mesure des angles de contact a été réalisée entre chaque étape de fonctionnalisation ces résultats sont reportés dans la figure 5.

La figure 5 montre que :
- les angles de contact sont significativement modifiés par les traitements successifs de la surface et des molécules D qui la recouvrent,
- La fonction maléimidyle reste active une fois greffée à la molécule D sur la surface de titane,
- Il est possible de greffer de façon spécifique des molécules porteuses de thiols sur ces surfaces.

### Exemple 15 : Mode de recouvrement de surface de verre.

Les surfaces sont oxydées avant traitement par les molécules gem-bisphosphoniques comme décrit dans par Hanson et Al. (2003, JACS, 125, 16074-80). Les plaques de verres (lames pour microscopie optique) sont immergées dans un mélange H₂SO₄ cc / H₂O₂ 35%, 1/3 v/v pendant 45 minutes à 50°C dans un bécher. Les plaques sont ensuite rincées à l'eau distillée. Ces plaques sont alors immergées dans une solution HCl 37% / H₂O₂ 35% (1/1 v/v) pendant 15 minutes à température ambiante. Les échantillons sont conservés dans l'eau. Les lames sont entièrement immergées dans une solution de molécule A (10⁻³M) à température ambiante sans agitation. Après trois heures, elles sont prélevées avec précaution et rincée par immersion dans l'acétone. La fixation par déshydratation du recouvrement est effectuée par lyophilisation à une température de 50°C sous un pression de 0.3 mbar pendant 15 heures.

Un échantillon référence a été réalisé sans molécule de recouvrement (Référence).

Tous ces supports subissent finalement un lavage aux ultrasons afin d'éliminer les potentielles multicouches ainsi que les molécules non fixées sur la surface.

Ces lavages sont réalisés comme suit :
- les supports sont plongés dans un large excès de solvant,
- sonication 15 minutes, élimination du solvant (2 fois),
- séchage des supports à l'étuve.

### Exemple 16 : Mesure de l'efficacité de fonctionnalisation par mesures des angles de contact et calcul des tensions superficielles de supports en verre

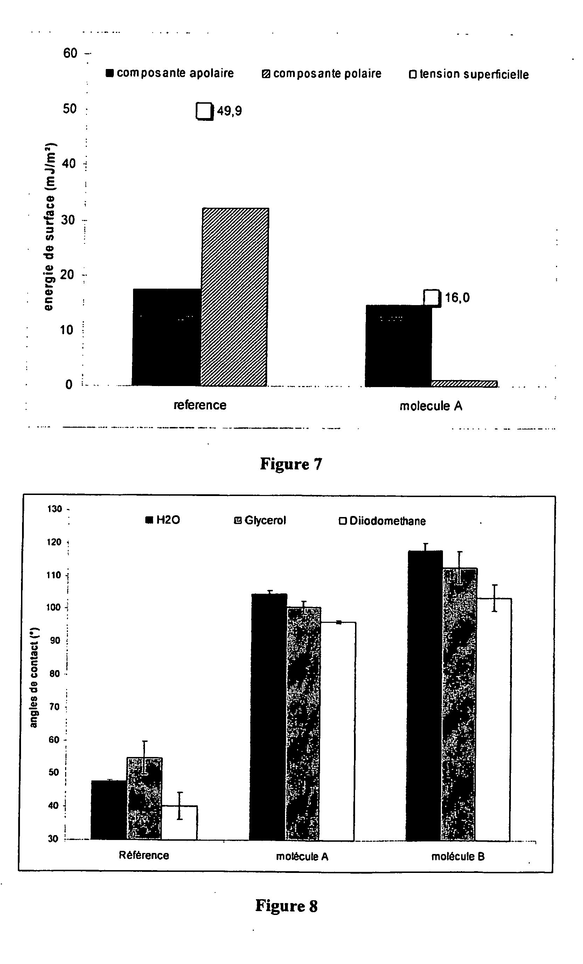

Le même protocole d'analyse de surface que dans l'exemple 10 est appliqué aux supports de verre recouverts des molécules A selon le procédé décrit à l'exemple 15.
- les angles de contacts et les tensions superficielles sont modifiés de façon significative (p<0.01) avec ces trois liquides vis à vis d'un support non traité (figure 6 et 7).
- les BP modifient les supports et que la déshydratation assure une fixation durable ;
- il est possible de greffer des molécules de type BP sur du verre de façon durable et résistante à un nettoyage par ultrasons.

### Exemple 17 Efficacité de la molécule B à limiter les tensions interfaciales sur des supports en acier inoxydable

Des supports en acier inoxydables 316 sont plongés dans une solution de la molécule A ou de la molécule B à 10⁻³ M pendant 3 heures. A l'issue de cette phase, les échantillons sont lavés dans l'acétone aux ultrasons pendant 10 minutes. Des échantillons références sont réalisés par trempage dans l'acétone (Référence)

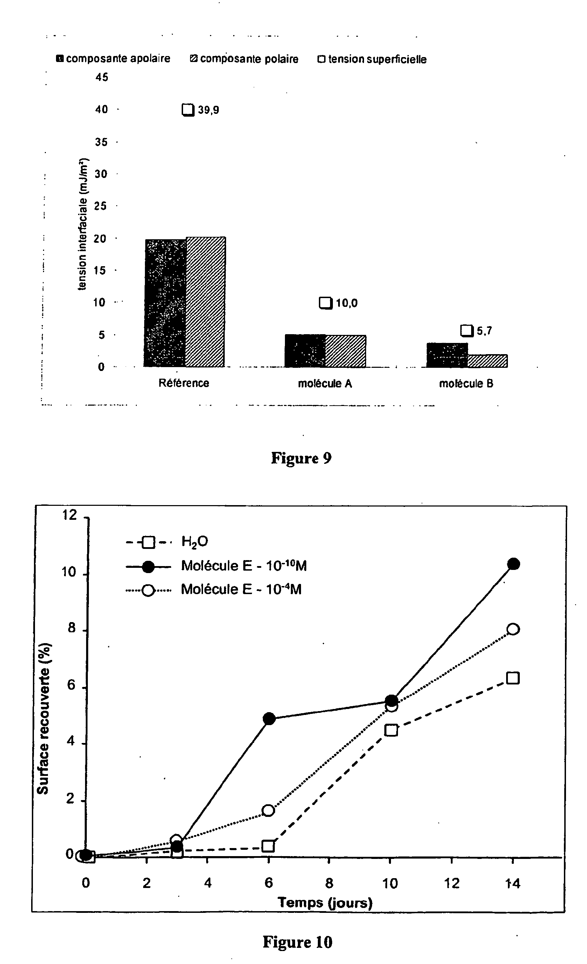

Le même protocole d'analyse de surface que dans l'exemple 10 est appliqué aux supports d'acier inoxydables recouverts des molécules A ou B. Ces mesures permettent de déterminer que :
- les angles de contacts sont modifiés de façon significative (p<0.01) avec ces trois liquides vis à vis d'un support non traité (figure 8).
- les tensions interfaciales (figure 9) montrent un très net abaissement des interactions entre les surfaces lorsque celles-ci sont recouvertes par des BP fluorés (molécules A et B). Celles-ci présentent les caractéristiques de lubrifiants de surface efficaces.

### Exemple 18 : Mode de recouvrement de surfaces de mica

Des plaques de mica fraîchement clivées sont plongées dans une solution de la molécule B (10⁻³M) pendant 3 heures à température ambiante et sans agitation. Les plaques sont ensuite prélevées délicatement et déshydratées à l'étuve pendant 15h. L'opération est reproduite au moins 1 fois.

### Exemple 19 : Mesure de l'efficacité de fonctionnalisation de supports en mica par mesures des angles de contact d'une goutte d'eau.

L'estimation de la fixation des molécules B sur le mica est effectuée de façon qualitative par mesure des angles de contact entre les surfaces modifiées et l'eau distillée. Ces mesures sont comparées avec celles réalisées sur le support avant le traitement par ces produits.

La modification de la surface traitée par les BP est représentée ci-dessous.

| | Référence | Molécule B |
|---|---|---|
| Angles de contact | < 5° | 85,5°+/-6,4° |

### Exemple 20 _{:} Prolifération de cellules de calvaria de souris sur des surfaces en titane traitées ou non par la molécule E.

Le test de prolifération est réalisé avec les cellules de calvaria de souris. Un millilitre d'une suspension homogène de cellules de calvaria (15 x 10⁻³ cellules/ml) est déposé délicatement sur chaque disque de Titane recouvert au préalable selon le protocole décrit dans l'exemple 11. Les plaques sont ensuite placées dans un incubateur à 37°C dans une atmosphère contenant 5% de CO2. Après 96 heures de culture les disques de Titane sont retirés des puits, rincés et déposés dans 500µl solution de trypsine-EDTA puis 1ml de milieu de culture (αMEM + 10% de SVF + 1% d'antibiotiques) est ajouté. Après centrifugation le culot cellulaire est repris dans 500µl de milieu et les comptages sont effectués à l'aide d'une cellule de Malassez et de bleu de trypan.

Le test de viabilité cellulaire est réalisé sur les cellules de calvaria de souris. Les pastilles de titane sont traitées comme précédemment. Après 96 heures de culture, le surnageant ainsi que les disques de Titane sont retirés des puits, et mis dans une nouvelle plaque. Le réactif XTT (Roche ; n° de lot : 13484900) est alors ajouté au milieu. Une lecture à 4 heures est réalisée sur un lecteur de plaque VICTOR 2, 1420 multilabel counter, PERKIN ELMER. L'intensité de coloration est mesurée en spectrophotométrie à 470nm.
- Le traitement par la molécule E permet dans ces conditions de culture d'obtenir un facteur de prolifération de 1,84. Dans le même temps, des pastilles traitées uniquement par de l'eau (témoin) ont un facteur de prolifération de 1,18.
- Morphologiquement, les pastilles traitées par la molécule E sont plus étalées et présentent des capacités d'adhérence supérieures au témoin.
- Le test de viabilité au XTT ne montre pas de différences significatives entre les échantillons témoin et ceux recouverts par la molécule E.

### Exemple 21 : Minéralisation de surface en titane traitées ou non par la molécule E par des cellules osteoblast-like (SaOS2)

Trois lots de disques de Titane sont recouverts selon le protocole décrit dans l'exemple 11 par des solutions aqueuses de la molécule E à 10⁻¹⁰M, 10⁻⁴ M et par de l'eau. Les échantillons ont été ensemencés par dépôt de 40 µL d'une suspension cellulaire de SaOS-2 à une densité de 2.10⁴ cellules/cm². Les ostéoblastes sont laissés à adhérer environ 3 heures avant l'ajout de 1 mL de milieu de culture DMEM contenant 1 % de L-glutamine, 2 % de pénicilline/streptomycine, et 10 % de sérum de veau foetal pendant une semaine. Le milieu est changé tous les 2 à 3 jours. Au bout de 7 jours de culture, le milieu standard de culture est remplacé par le milieu de minéralisation comprenant 10 mM de β-glycérophosphate (Sigma) et 50 µg/mL d'acide ascorbique (Sigma), considérant ce jour comme J₀ de la minéralisation.

A J₃, J₆, J₁₀ et J₁₄ les échantillons sont incubés avec de la calcéïne (25 µg.mL⁻¹) pendant 2 heures, qui met en évidence la présence de sels de calcium. Les échantillons ont ensuite été fixés au paraformaldéhyde 4 % (Sigma) pendant 20 minutes. Après avoir été rincé plusieurs fois au PBS, la minéralisation est observée en microscopie à fluorescence à 488 nm (Leica). La surface recouverte par la minéralisation est mesurée par analyse d'images, avec le logiciel ImageJ 1.34s (National Institutes of Health, USA). Après seuillage, le nombre de pixels fluorescents a été quantifié et comparé au nombre de pixels total pour déterminer le pourcentage de recouvrement par échantillon. Cinq images par échantillon au grossissement x100 ont été analysées.

Dans les 3 groupes, la minéralisation a commencé au troisième jour. Au bout de 6 jours, les dépôts minéralisés sont plus abondants sur le titane recouvert pas la molécule E comparé au titane seul. En revanche, la minéralisation est plus rapide pour la concentration 10⁻¹⁰M que pour 10⁻⁴M. A 14 jours, la surface minéralisée était 1,5 fois plus importante sur les pastilles traitées par la molécule E à 10⁻¹⁰ M que sur celles traitées par de l'eau (figure 10).
Il peut donc être conclu que le dépôt de minéral se fait plus rapidement sur les pastilles recouvertes avec la molécule E que sur le titane seul. La minéralisation est plus abondante pour une surface recouverte par la molécule E à 10⁻¹⁰M que 10⁻¹⁰M.

### Exemple 22 : Propriété d'anticontamination bactérienne de surface d'hydroxyapatite (HAP) traitées ou non par la molécule F

Les surfaces HAP sont traitées par une solution contenant des molécules gem-bisphosphoniques. Un tube témoin est traité à l'eau distillée stérile. Le recouvrement de la surface HAP est réalisé à 37°C pendant 3 minutes. Un volume de milieu nutritif (Hutteau et Mathlouti, 1998) est ensuite ajouté, suivi immédiatement par une suspension bactérienne de *Streptococcus mutans.* Les tubes sont incubés de 8 ou 24 heures à 37°C sous légère agitation. A chaque point de la cinétique, après élimination du surnageant et trois cycles de rinçage, les bactéries adhérentes à la HAP sont détachées par ultrasons. La suspension bactérienne ainsi obtenue est dénombrée par dilutions successives et étalement sur gélose au sang.

Les résultats obtenus sont présentés dans le tableau ci-dessous, ils sont exprimés en réduction décimale de la contamination par rapport à une surface non traitée par la molécule F.

| | 8 heures | 24 heures |
|---|---|---|
| Réduction de contamination | -1,85 ± 0,29 Log | -2,77 ± 0,73 Log |

## Revendications

1. Procèdé de recouvrement d'un substrat métallique ou minéral par une couche moléculaire de fonctionnalisation, **caractérisé en ce qu'**il comprend les étapes successives suivantes:
a) oxydation préalable de la surface du substrat si celui- ci n'est pas déjà au moins partiellement hydroxylé de façon à disposer de fonctions hydroxyles à la surface du substrat,
b) mise en contact de la surface du substrat avec une composition liquide, gazeuse ou supercritique de recouvrement contenant des composes gem-bisphosphoniques, et/ou leurs sels toxico logiquement acceptables, jusqu'à autoassemblage desdites composés gem-bisphosphoniques en une couche recouvrant ladite surface,
c) élimination de ladite composition liquide, gazeuse ou supercritique de recouvrement,
d) déshydratation thermique de la surface ainsi recouverte.

2. Procède de recouvrement selon la revendication 1, **caractérisé en ce qu'**il n'est pas réalise dans des conditions anhydres.

3. Procède de recouvrement selon l'une quelconque des revendications 1 à 2, **caractérisé en ce que** la mise en contact de la composition de recouvrement avec, la surface du substrat est remisée par trempage du substrat dans une solution aqueuse contenant entre 0,001 % et 5% de composés gem-bisphosphoniques, pendant un temps compris entre 5 minutes et 3 heures, à température ambiante, avec ou sans agitation.

4. Procède de recouvrement selon l'une des revendications 1 à 3, **caractérisé en ce que** la déshydration de la surface du substrat est réalisée par chauffage de celle-çi à une température comprise entre 20°C et 150°C, de préférence à environ 50°C, sous une pression comprise entre 0,01 mBar et 1 Bar, de préférence à 0,3 mbar, pendant un temps compris entre 1 et 72 heures, de préférence pendant environ 15 heures.

5. Procède de recouvrement selon l'une quelconque des revendications 1 à 4, **caractérisé en ce que** les composés bisphosphoniques répondent à la formule (I) suivante: 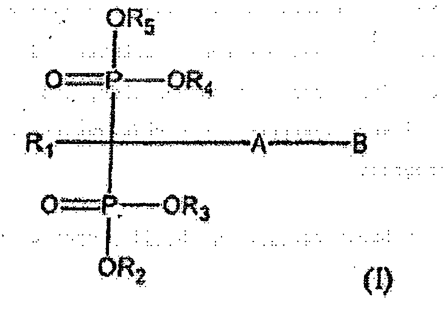 dans laquelle :
- R2, R3, R4, R5 représentent indépendamment l'un de l'autre un atome d'hydrogène ou un groupe alkyle en C1-C10, tel que méthyle ou éthyle ; de préférence R2, R3, R4, R5 sont identiques et représentent un atome d'hydrogène ;
- A représente une liaison chimique simple, -O-, -S- ou -NH-, de préférence une liaison chimique simple;
- B représente:
a) un groupement alkyle en C1-C100, linéaire ou ramifié, saturé ou non, la chaîne alkyle pouvant être substituée ou interrompue par 1 à 10 groupements aryles ; ou
b) un groupement -(OH₂)m-X dans lequel m est un entier compris entre 1 et 100 et X est un groupement alkyle en C1-C100 saturé ou non, pouvant être perfluoré ou partiellement fluoré, la chaîne alkyle pouvant être substituée ou interrompue par 1 à 10 groupements aryle pouvant être perfluorés ou non;
de préférence B représente un groupement alkyle en C9-C20 perfluoré ou partiellement fluoré; et
- R1 représente un atome d'hydrogène ou un groupement -OH, -NH₂, alkyle en C1-C100, pouvant être perfluoré ou partiellement fluoré; R1 représente de préférence un groupement -OH,
ainsi que leurs sels toxicologiquement acceptables,
ou bien à la formule (I') suivante : 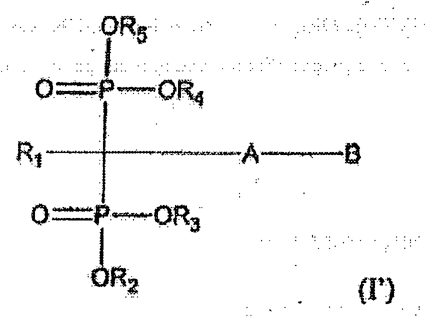 dans laquelle :
- B représente un groupement -(CH₂)ₘ-D-E dans lequel :
- m est un entier compris entre 1 et 100,
- D est une liaison chimique simple ou un groupement -O-, -S-, -OCO-, -COO-, - COS-, -SCO-, -SCS-, -CONH-, -HNCO-, -HNCO-CF(CF₃)- ou -NH- CO-NH-NH-CS-NH-, et
- E est un groupement; -(O-CF₂-CF₂)ₙ-OR_{f}, -(O-CF₂-CF(CF₃))ₙ-OR_{f}, -(O-CF(CF₃)-CF₂)ₙ-OR_{f} ou -(O-CF₂-CF₂-CF₂)ₙ-OR_{f}, où n est un entier compris entre 1 et 100 et R_{f} est un groupement alkyle perfluoré en C1-C10, ou bien un groupement : 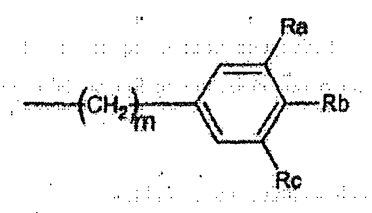 dans lequel, Ra, Rb, Rc représentent indépendamment l'un de l'autre un atome d'hydrogène, un alkyle en C1-C100 perfluoré ou un groupement: -(O-CF₂-CF₂)ₙ-OR_{f}, -(O-CF₂-CF(CF₃)ₙ-OR_{f}; -(O- CF(CF₃)-CF₂)ₙ-OR_{f} ou -(Q-CF₂-CF₂-CF₂)ₙ-OR_{f}, où n et R_{f} sont tels que définis précédemment et m est un entier compris entre () et 20,
- A représente :
a) une liaison chimique simple, ou
b) une fonction -O-, -S- ou -NH-, et
- R₁, R₂, R₃, R_{4 e}t R₅ sont tels que définis précédemment, ainsi que leurs sels toxico logiquement acceptables.

6. Procédé de recouvrement selon la revendication 5, pour modifier les propriétés de mouillabilité, de lubrification ou d'adhésion de surfaces métalliques ou minérales, pour limiter la formation de givre ou l'étalement de liquide sur ces surfaces notamment en favorisant l'effet épilame, ou pour protéger ces surfaces contre l'adhésion des graffitis, ou pour faciliter le glissement de pièces mécaniques.

7. Procédé de recouvrement selon l'une quelconque des revendications 1 à 4, **caractérisé en ce que** les composés gem-bisphosphoniques répondent à la formule (II) suivante : 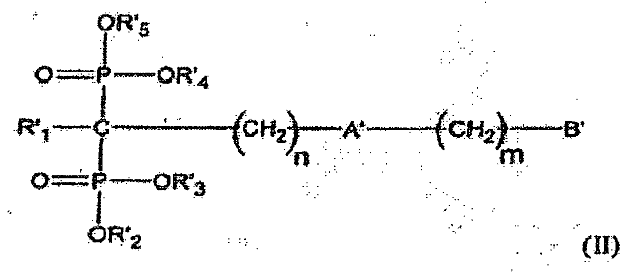 dans laquelle :
- n et m sont des entiers compris indépendamment l'un de l'autre entre 0 et 20; de préférence n = 3, 4 ou 5 et = 5 ;
- R' 1 représente un atome d'hydrogène, un groupement -OH, une aminé, ou un groupement alkyle en C1-C10, éventuellement fluoré; de préférence, R'1 représente un groupement -OH :
- R'2, R'3, R'4, R'5 représentent indépendamment l'un de l'autre un atome d'hydrogène ou un groupement alkyle en C1-C10, tel que méthyle ou éthyle ; de préférence R'2, R'3, R'4, R'5 sont identiques et représentent un atome d'hydrogène ;
- A' représente un groupement -L-Y- où :
L est une liaison chimique simple, un -OOC-, -CO-, CONH-, -HNCO-, -NH-CO-NH-, -NH-, -COS-, -SCO-, -SCS- ou -NH-CS-NH- ; et
Y est :
a. une liaison chimique simple;
b. un groupement de formule 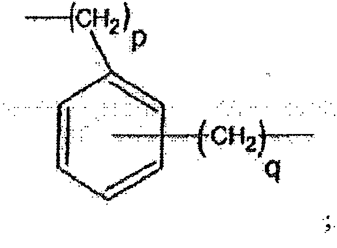 ou
c. un groupement de formule 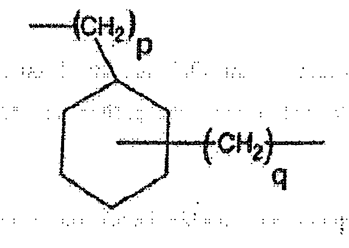 ou
d. un groupement de formule 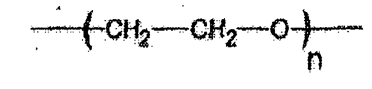 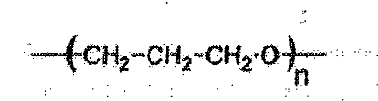
dans lesquels, n, p et q représentent indépendamment l'un de l'autre des entiers compris entre 0 et 100 ; et
- B' représente :
a) un groupement de formule 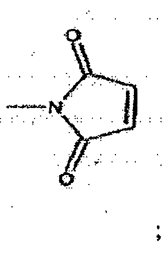 ou
b) un groupement de formule 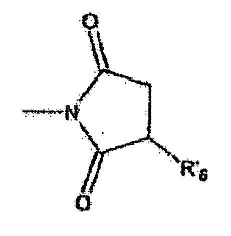
dans lequel R' 6 représente un acide animé, un peptide linéaire ou cyclique, notamment un peptide CRGD ou RGDC, une protéine, une glyco-protéine, un acide nucléique simple ou double brin, un antibiotique, une hormone, un saccharide ou polysaccharide, une vitamine, un anticorps, un fragment d'anticorps,
un haptène, un facteur de croissance, une cytokine, ou un radical pharmaceutique actif ; de préférence, R' 6 représente un acide aminé ou un peptide, tel que CRCD ou RGDC, ainsi que leurs sels toxicologiquement acceptables.

8. Procédé de recouvrement selon la revendication 7, pour le greffage de molécules biologiquement actives sur des surfaces métalliques ou minérales, pour notamment augmenter l'adhésion d'ostéoblastes sur des prothèses orthopédiques ou implants dentaires, pour favoriser la minéralisation, pour modifier l'équilibre des activités ostéoblastiques et ostéoclastiques, ou pour fonctionnaliser des surfaces de silice pour la purifïcation, l'adhésion ou la reconnaissance d'entités biologiques.

9. Procédé de recouvrement selon l'une quelconque des revendications 1 à 4, **caractérisé en ce que** les composés gem-bisphosphoniques répondent à la formule (III) suivante : 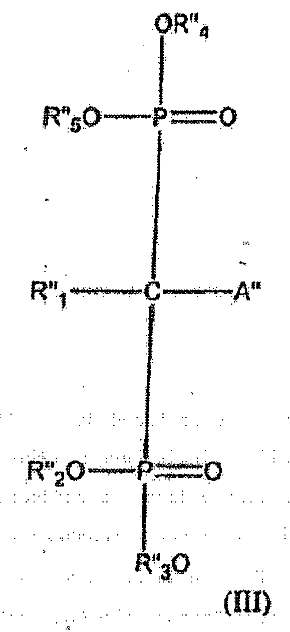 dans laquelle :
- R"1 représente un atome d'hydrogène, un groupe -OH, -NH₂, un groupe alkyle en C1-C10 éventuellement fluoré ;
- R"2, R"3, R"4, R"5 représentent indépendamment l'un de l'autre un atome d'hydrogène ou un groupe alkyle en C1-C10, tel que méthyle ou éthyle ; de préférence R"2, R"3, R"4, R"5 sont identiques et représentent un atome d'hydrogène ; et
- A" représente :
a un groupement de formule
-CH₂O-(CH₂CH₂O)ₙ-X
dans lequel n est un entier compris entre 0 et 100 et X représente un hydrogène ou un groupement alkyle en C1-C5 ; de préférence X représente un groupement méthyle ; ou
b. un groupement de formule 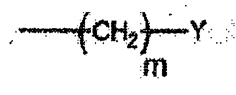
dans lequel m est un entier compris entre 0 et 20, de préférence compris entre 3 et 6, et Y représente un groupement -NH-CO-CH₃, -CO-NH-CH₃-N(CH₃)-CO-CH₃ ou -CO-N(CH₃)₂,
ainsi que leurs sels toxicologiquement acceptables.

10. Procédé de recouvrement selon la revendication 9, pour empêcher ou limiter la fixation de macro molécules, de microorganismes ou de biofilms sur des surfaces métalliques ou minérales.

11. Substrat fonctionnalisé susceptible d'être obtenu par le procède selon la revendication 5.

12. Utilisation du substrat fonctionnalisé selon la revendication 11, dans des pièces mécaniques, notamment destinées à l'horlogerie; l'industrie automobile ou aéronautique, ou bien dans des microsystèmes électromécaniques ou nanosystèmes électromécaniques, pour limiter les forces de collage, faciliter le glissement entre deux pièces mécaniqes, limiter l'étalement de liquide sur ces surfaces en favorisant l'effet épilame ou limiter la formation de givre.

13. Composés gem-bisphosphoniques répondant à la formule (Ia) suivante : 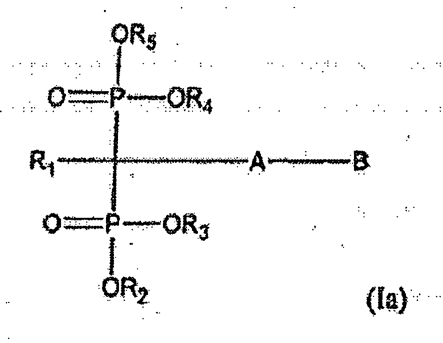 dans laquelle : - R2, R3, R4, R5 représentent indépendamment l'un de l'autre un atome d'hydrogène ou un groupe alkyle en C1-C10, tel que méthyle ou éthyle ; de préference R2, R3, R4, R5 sont identiques et représentent un atome d'hydrogène ;
- A représente une liaison chimique simple, -O-, -S- ou -NH-, de préférence une liaison chimique simple;
- B représente un groupement -(CH₂)m-X dans lequel m est un entier compris entre 1 et 100 et X est un groupement alkyle en C1-C100 saturé ou non, pouvant être perfluoré ou partiellement fluoré, la chaîne alkyle pouvant être substituée ou interrompue par 1 à 10 groupements aryles pouvant être perfluorés ou non;
de préférence, m est compris entre 1 et 20 et X représente un groupement alkyle perfluoré ou partiellement fluoré en C9-C20 ; de manière encore plus préférée, B représente un groupement alkyle en C9-C20 perfluoré ou partiellement fluoré; et
- R1 représente un atome d'hydrogène ou un groupement -OH, -NH₂; alkyle en C1-C100, pouvant être complètement ou partiellement fluoré ; R1 représente de préférence un groupement -OH ;
ainsi que leurs sels toxicologiquement acceptables.

14. Substrat fonctionnalisé susceptible d'être obtenu par le procédé selon la revendication 6.

15. Utilisation du substrat fonctionnalisé selon la revendication 14, dans des prothèses orthopédiques et des implants dentaires, pour augmenter l'adhésion d'ostéoblastes, favoriser la minéralisation ou modifier l'équilibre des activités ostéoblastiques et ostéoclastiques sur ces prothèses ou implants.

16. Utilisation du substrat fonctionnalisé selon la revendication 14, pour le greffage de molécules bio logiquement actives sur des surfaces métalliques ou minérales, ou pour fonctionnaliser des surfaces pour la purification, l'adhésion; ou la reconnaissance d'entités biologiques.

17. Composés gem-bisphosphoniques répondant à la formule (IIa) suivante : 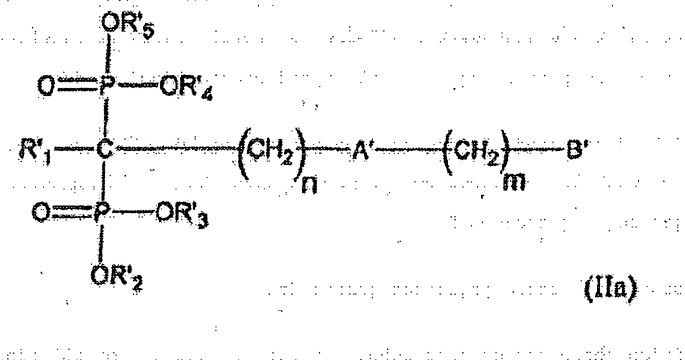 dans laquelle :
- n et m sont des entiers compris indépendamment l'un de l'autre entre 0 et 20; de préférence, n = 3, 4 ou 5 et m = 5 ;
- R' 1 représente un atome d'hydrogène, un groupement -OH, une aminé ramifiée ou non, ou un groupement alkyle en C1-C10, éventuellement fluoré, de préférence R' 1 représente un groupement -OH ;
- R'2, R'3, R'4, R'5 représentent indépendamment l'un de l'autre un atome d'hydrogène ou un groupement alkyle en C1-C10, tel que méthyle ou éthyle ; de préférence R'2, R'3, R'4, R'5 sont identiques et représentent un atome d'hydrogène
- A' représente un groupement -L-Y- où :
L est une liaison chimique simple, un groupement -O-, -S-, -OOC-, -COO-, - CONH-, -HNCO-, -MH-CO-NH-, -NH- ; et
Y est :
a. une liaison chimique ;
b. un groupement de formule 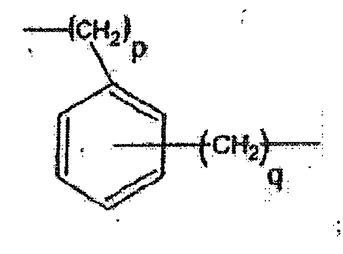 ou
c. un groupement de formule 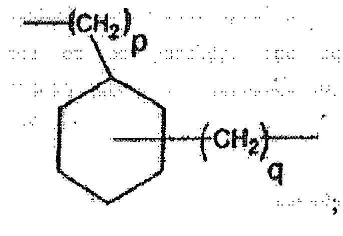 ou
d. un groupement de formule 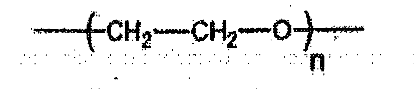 ou 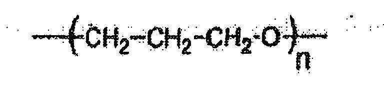
dans lesquels n, p et q représentent indépendamment l'un de l'autre des entiers compris entre 0 et 100 ; de préférence compris entre 0 et 10 ;
de préférence, A' représente un groupement -HNCO- ; et
- B' représente un groupement de formule 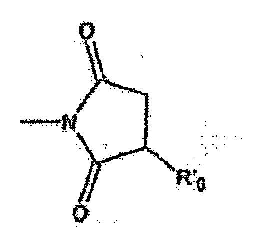
dans lequel R'6 représente un acide aminé, un peptide linéaire ou cyclique, notamment un peptide CRGD ou RGDC, une protéine, une glyco -protéine, un acide nucléique simple ou double brin, un antibiotique, une hormone, un saccharide ou polysaccharide, une vitamine, un anticorps, un fragment d'anticorps, un haptène, un facteur de croissance, une cytokine, ou un radical pharmaceutiquement actif de préférence R'6 représente un peptide CRGD ou RGDC;
ainsi que leurs sels toxicologiquement acceptables.

18. Substrat fonctionnalisé susceptible d'être obtenu par le procédé selon la revendication 9.

19. Utilisation des composés gem-bisphosphoniques de formule (III) tels que définis à la revendication 9, contre la biocontamination de surfaces métalliques ou minérales ou comme agent de biocompatibilité, notamment pour empêcher ou limiter la fixation de macromolécules, des microorganismes, de préférence des bactéries, ou le développement du biofilm sur des surfaces métalliques ou minérales.

20. Utilisation des composés de formule (III) selon la revendication 19, **caractérisés en ce que** les surfaces métalliques appartiennent au groupe comprenant le fer, l'acier inoxydable, le chrome, l'aluminium, le zinc, le titane, le tungstène, le plomb ou le cuivre, ainsi que les oxydes, alliages ou composites contenant au moins l'un de ces métaux et les surfaces minérales appartiennent au groupe comprenant le silicium et ses dérivés, les matériaux silicieux, les surfaces calciques, les surfaces céramiques ou dentaires.

21. Utilisation des composés de formule (III) selon la revendication 19 ou 20, **caractérisée en ce que** les surfaces sont la surface de matériel industriel, agroalimentaire ou hospitalier, de bâtiments, de constructions ou de véhicules terrestres, aériens ou maritimes, de matériel de climatisation ou de réfrigération ou bien la surface d'instruments chirurgicaux, de prothèses, d'instruments de dentisterie ou de capteurs biologiques et médicaux.

## Claims

1. Method of covering a metal or inorganic substrate by a molecular functionalization layer, **characterized in that** it comprises the following successive steps:
a) prior oxidation of the surface of the substrate if it is not already at least partially hydroxylated so as to create hydroxyl functions on the surface of the substrate,
b) contact of the surface of the substrate with a liquid, gaseous or supercritical coating composition containing gem-bisphosphonic compounds, and/or the toxicologically acceptable salts of same, until self-assembly of said gem-bisphosphonic compounds in a layer covering said surface,
c) removal of said liquid, gaseous or supercritical coating composition,
d) thermal dehydration of the surface thus covered.

2. The method of covering according to claim 1, **characterized in that** it is not carried out under anhydrous conditions.

3. The method of covering according to either of claims 1 or 2, **characterized in that** the contact of the coating composition with the surface of the substrate is carried out by dipping the substrate in an aqueous solution containing between 0.001% and 5% of gem-bisphosphonic compounds, for a period of time ranging between 5 minutes and 3 hours, at room temperature, with or without stirring.

4. The method of covering according to any of claims 1 to 3, **characterized in that** the dehydration of the surface of the substrate is carried out by heating said surface at a temperature between 20 °C and 150 °C, preferably at approximately 50 °C, under a pressure between 0.01 mbar and 1 bar, preferably 0.3 mbar, for a period of time ranging between 1 hour and 72 hours, preferably for approximately 15 hours.

5. The method of covering according to any of claims 1 to 4, **characterized in that** the bisphosphonic compounds are of following formula (I): 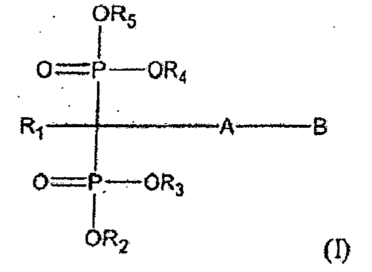 wherein:
- R₂, R₃, R₄, R₅ represent independently of one another a hydrogen atom or a C₁-C₁₀ alkyl group, such as methyl or ethyl; preferably R₂, R₃, R₄, R₅ are identical and represent a hydrogen atom;
- A represents a single chemical bond, -O-, -S- or -NH-, preferably a single chemical bond;
- B represents:
a) a C₁-C₁₀₀ alkyl group, linear or branched, saturated or unsaturated, the alkyl chain optionally substituted or interrupted by 1 to 10 aryl groups; or
b) a -(CH₂)ₘ-X group, wherein m is an integer between 1 and 100 and X is a C₁-C₁₀₀ alkyl group, saturated or unsaturated, optionally perfluorinated or partially fluorinated, the alkyl chain optionally substituted or interrupted by 1 to 10 aryl groups which may or may not be perfluorinated;
preferably B represents a perfluorinated or partially fluorinated C₉-C₂₀ alkyl group; and
- R₁ represents a hydrogen atom or an -OH, -NH₂, C₁-C₁₀₀ alkyl group, optionally perfluorinated or partially fluorinated; R₁ preferably represents an -OH group,
as well as toxicologically acceptable salts of same,
or following formula (I'): 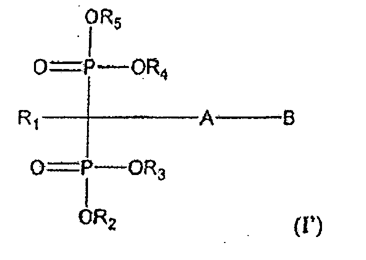 wherein:
- B represents a -(CH₂)ₘ-D-E group, wherein:
- m is an integer between 1 and 100,
- D is a single chemical bond or an -O-, -S-, - OCO-, -COO-, -COS-, -SCO-, -SCS-, -CONH-, -HNCO-, - HNCO-CF(CF₃)- or -NH-CO-NH-NH-CS-NH- group, and
- E is an -(O-CF₂-CF₂)ₙ-OR_{f}, -O-CF₂-CF(CF₃))ₙ-OR_{f}, -(O-CF(CF₃)-CF₂)ₙ-OR_{f} or -(O-CF₂-CF₂-CF₂)ₙ-OR_{f} group, wherein n is an integer between 1 and 100 and R_{f} is a perfluorinated C₁-C₁₀ alkyl group, or the following group: 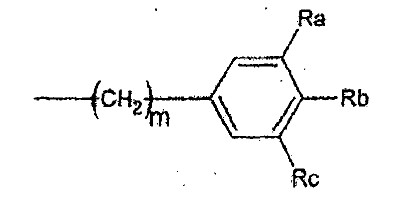 wherein, Ra, Rb, Rc represent independently of one another a hydrogen atom, a perfluorinated C₁-C₁₀₀ alkyl or an - (O-CF₂-CF₂)ₙ-OR_{f}, -(O-CF₂-CF(CF₃))ₙ-OR_{f}, -(O-CF(CF₃)-CF₂)ₙ-OR_{f} or -(O-CF₂-CF₂-CF₂)ₙ-OR_{f} group, wherein n and R_{f} are as previously defined and m is an integer between 0 and 20,
- A represents:
a) a single chemical bond, or
b) an -O-, -S- or -NH- function, and
- R₁, R₂, R₃, R₄ and R₅ are as previously defined, as well as toxicologically acceptable salts of same.

6. The method of covering according to claim 5, to modify the properties of wettability, lubrication or adhesion of metal or inorganic surfaces, to limit the formation of crystallization or the spreading of liquid on these surfaces in particular by supporting the epilame effect, or to protect these surfaces against the adhesion of graffiti, or to facilitate the sliding of machine parts.

7. The method of covering according to any of claims 1 to 4, **characterized in that** the gem-bisphosphonic compounds are of following formula (II): 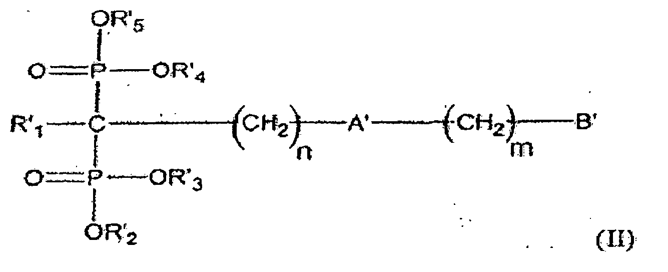 wherein:
- n and m are integers independent of one another between 0 and 20; preferably n=3, 4 or 5 and m=5;
- R'₁ represents a hydrogen atom, an -OH group, an amino, or a C₁-C₁₀ alkyl group, optionally fluorinated; preferably, R'₁ represent an -OH group;
- R'₂, R'₃, R'₄, R'₅ represent independently of one another a hydrogen atom or a C₁-C₁₀ alkyl group, such as methyl or ethyl; preferably R'₂, R'₃, R'₄, R'₅ are identical and represent a hydrogen atom;
- A' represents an -L-Y- group wherein:
L is a single chemical bond, an -O-, -S-, -OOC-, - COO-, -CONH-, -HNCO-, -NH-CO-NH-, -NH-, -COS-, -SCO-, - SCS- or -NH-CS-NH- group; and
Y is:
a) a single chemical bond;
b) a group of formula 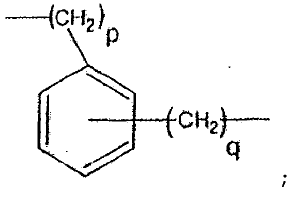 or
c) a group of formula 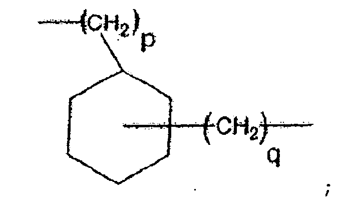 or
d) a group of formula 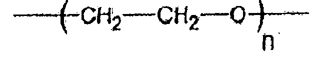 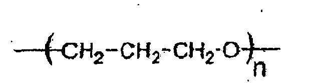
wherein, n, p and q represent independently of one another integers between 0 and 100; and
- B' represents:
a) a group of formula 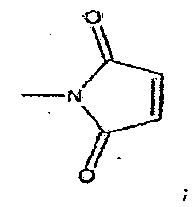 or
b) a group of formula 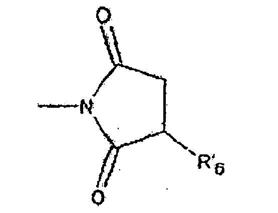
wherein R'₆ represents an amino acid, a linear or cyclic peptide, in particular a CRGD or RGDC peptide, a protein, a glycoprotein, a single or double strand nucleic acid, an antibiotic, a hormone, a saccharide or polysaccharide, a vitamin, an antibody, an antibody fragment, a hapten, a growth factor, a cytokine, or a pharmaceutically active radical; preferably, R'₆ represents an amino acid or a peptide, such as CRGD or RGDC, as well as toxicologically acceptable salts of same.

8. The method of covering according to claim 7, for the grafting of biologically active molecules on metal or inorganic surfaces, in particular to increase the adhesion of osteoblasts on orthopedic prostheses or dental implants, to favour mineralization, to modify the equilibrium of osteoblastic and osteoclastic activities, or to functionalize silica surfaces for the purification, adhesion or recognition of biological entities.

9. The method of covering according to any of claims 1 to 4, **characterized in that** the gem-bisphosphonic compounds are of following formula (III): 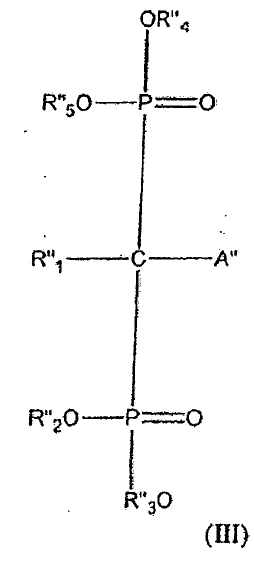 wherein:
- R"₁ represents a hydrogen atom, an -OH group, - NH₂, a C₁-C10 alkyl group, optionally fluorinated;
- R"₂, R"₃, R"₄, R"₅ represent independently of one another a hydrogen atom or a C₁-C₁₀ alkyl group, such as methyl or ethyl; preferably R"₂, R"₃, R"₄, R"₅ are identical and represent a hydrogen atom; and
- A" represents:
a) a group of formula
-CH₂O-(CH₂CH₂O)ₙ-X
wherein n is an integer between 0 and 100 and X represents a hydrogen or a C₁-C₅ alkyl group; preferably X represents a methyl group; or
b) a group of formula 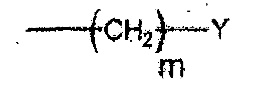
wherein m is an integer between 0 and 20, preferably between 3 and 6, and Y represents a -NH-CO-CH₃, -CO-NH-CH₃-N(CH₃)-CO-CH₃ or -CO-N(CH₃)₂ group,
as well as the toxicologically acceptable salts of same.

10. The method of covering according to claim 9, to prevent or limit the binding of macromolecules, microorganisms or biofilms on metal or inorganic surfaces.

11. A functionalized substrate likely to be obtained by the method according to claim 5.

12. Use of the functionalized substrate according to claim 11, in mechanical parts, in particular in watch- and clock-making, the automobile or aeronautical industry, or in electromechanical microsystems or nanosystems, to limit sticking forces, to facilitate sliding between two mechanical parts, to limit the spreading of liquid on these surfaces by supporting the epilame effect or to limit the formation of crystallization.

13. Gem-bisphosphonic compounds of following formula (Ia) : 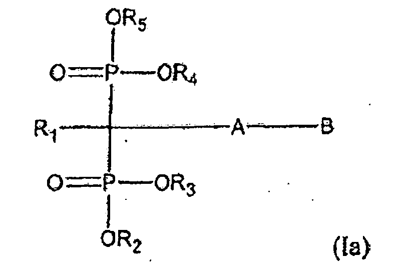 wherein: R₂, R₃, R₄, R₅ represent independently of one another a hydrogen atom or a C₁-C₁₀ alkyl group, such as methyl or ethyl; preferably R₂, R₃, R₄, R₅ are identical and represent a hydrogen atom;
- A represents a single chemical bond, -O-, -S- or -NH-, preferably a single chemical bond;
- B represents a -(CH₂)ₘ-X group wherein m is an integer between 1 and 100 and X is a C₁-C₁₀₀ alkyl group, saturated or unsaturated, optionally perfluorinated or partially fluorinated, the alkyl chain optionally substituted or interrupted by 1 to 10 aryl groups optionally perfluorinated or not;
preferably, m is between 1 and 20 and X represents a perfluorinated or partially fluorinated C₉-C₂₀ alkyl group; more preferably, B represents a perfluorinated or partially fluorinated C₉-C₂₀ alkyl group; and
- R₁ represents a hydrogen atom or an -OH, -NH₂, C₁-C₁₀₀ alkyl group, optionally completely or partially fluorinated; preferably R₁ represents an -OH group;
as well as the toxicologically acceptable salts of same.

14. A functionalized substrate likely to be obtained by the method according to claim 6.

15. Use of the functionalized substrate according to claim 14, in orthopedic prostheses and dental implants, to increase the adhesion of osteoblasts, to favour mineralization or to modify the equilibrium of osteoblastic and osteoclastic activities on these prostheses or implants.

16. Use of the functionalized substrate according to claim 14, for grafting biologically active molecules on metal or inorganic surfaces, or to functionalize surfaces for the purification, adhesion or recognition of biological entities.

17. Gem-bisphosphonic compounds of following formula (IIa): 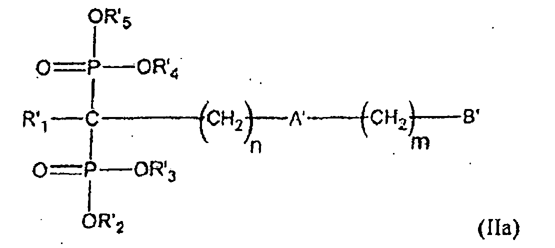 wherein:
- n and m are integers independent of one another between 0 and 20; preferably, n=3, 4 or 5 and m=5;
- R'₁ represents a hydrogen atom, an -OH group, a branched or linear amino, or a C₁-C₁₀ alkyl group, optionally fluorinated; preferably R'₁ represents an - OH group;
- R'₂, R'₃, R'₄, R'₅ represent independently of one another a hydrogen atom or a C₁-C₁₀ alkyl group, such as methyl or ethyl; preferably R'₂, R'₃, R'₄, R'₅ are identical and represent a hydrogen atom;
- A' represents an -L-Y- group wherein:
L is a single chemical bond, an -O-, -S-, -OOC-, - COO-, -CONH-, -HNCO-, -NH-CO-NH-, -NH- group; and
Y is:
a) a chemical bond;
b) a group of formula 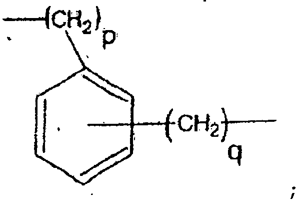 or
c) a group of formula 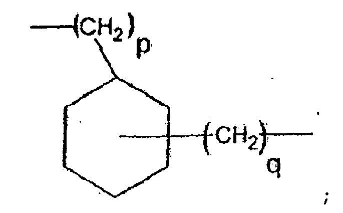 or
d) a group of formula 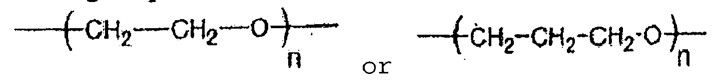
wherein n, p and q represent independently of one another integers between 0 and 100; preferably between 0 and 10;
preferably, A' represents an -HNCO- group; and
- B' represents a group of formula 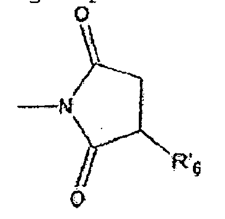
wherein R'₆ represents an amino acid, a linear or cyclic peptide, in particular a CRGD or RGDC peptide, a protein, a glycoprotein, a single or double strand nucleic acid, an antibiotic, a hormone, a saccharide or polysaccharide, a vitamin, an antibody, an antibody fragment, a hapten, a growth factor, a cytokine, or a pharmaceutically active radical, preferably R'₆ represents a CRGD or RGDC peptide;
as well as the toxicologically acceptable salts of same.

18. A functionalized substrate likely to be obtained by the method according to claim 9.

19. Use of gem-bisphosphonic compounds of formula (III) as defined in claim 9, against the biocontamination of metal or inorganic surfaces or as an agent of biocompatibility, in particular to prevent or limit the binding of macromolecules or microorganisms, preferably bacteria, or the development of biofilm on metal or inorganic surfaces.

20. Use of compounds of formula (III) according to claim 19, **characterized in that** the metal surfaces belong to the group that includes iron, stainless steel, chromium, aluminum, zinc, titanium, tungsten, lead or copper, as well as oxides, alloys or composites containing at least one of these metals and inorganic surfaces belonging to the group that includes silicon and derivatives of same, siliceous materials, calcic surfaces, ceramic or dental surfaces.

21. Use of compounds of formula (III) according to claim 19 or 20, **characterized in that** the surfaces are those used in factories, food processing, hospitals, buildings, construction or land, air or sea vehicles, air-conditioning or refrigeration equipment or the surface of surgical instruments, prostheses, dental instruments or biological and medical sensors.

## Patentansprüche

1. Verfahren zur Bedeckung eines metallischen oder mineralischen Substrats durch eine funktionalisierende Molekularschicht, **dadurch gekennzeichnet, dass** es die folgenden aufeinander folgenden Schritte umfasst:
a) vorheriges Oxidieren der Fläche des Substrats, wenn dieses nicht bereits mindestens teilweise hydroxyliert ist, um auf der Fläche des Substrats über Hydroxylfunktionen zu verfügen,
b) In-Kontakt-Bringen der Fläche des Substrats mit einer flüssigen, gasförmigen oder superkritischen Bedeckungszusammensetzung, enthaltend Gem-Bisphonsäureverbindungen und/oder ihre toxikologisch annehmbaren Salze, bis zur Selbstassemblierung der Gem-Bisphonsäureverbindungen in eine Schicht, die die Fläche bedeckt,
c) Eliminieren der flüssigen, gasförmigen oder superkritischen Bedeckungszusammensetzung,
d) thermisches Entwässern der so bedeckten Fläche.

2. Verfahren zur Bedeckung nach Anspruch 1, **dadurch gekennzeichnet, dass** es nicht unter wasserfreien Bedingungen durchgeführt wird.

3. Verfahren zur Bedeckung nach einem der Ansprüche 1 bis 2, **dadurch gekennzeichnet, dass** das In-Kontakt-Bringen der Bedeckungszusammensetzung mit der Fläche des Substrats durch Eintauchen des Substrats in eine wässrige Lösung, enthaltend zwischen 0,001 % und 5 % Gem-Bisphonsäureverbindungen, während eines Zeitraums zwischen 5 Minuten und 3 Stunden bei Zimmertemperatur, mit oder ohne Rühren erfolgt.

4. Verfahren zur Bedeckung nach einem der Ansprüche 1 bis 3, **dadurch gekennzeichnet, dass** das Entwässern der Fläche des Substrats durch Erhitzen dieser auf eine Temperatur zwischen 20 °C und 150 °C, vorzugsweise auf ungefähr 50 °C, unter einem Druck zwischen 0,01 mBar und 1 Bar, vorzugsweise von 0,3 mBar, während eines Zeitraums zwischen 1 und 72 Stunden, vorzugsweise während ungefähr 15 Stunden, erfolgt.

5. Verfahren zur Bedeckung nach einem der Ansprüche 1 bis 4, **dadurch gekennzeichnet, dass** die Bisphonsäureverbindungen der folgenden Formel (I) entsprechen: 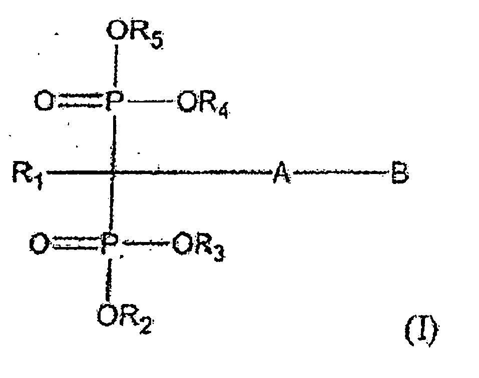 in der:
- R2, R3, R4, R5 unabhängig voneinander ein Wasserstoffatom oder eine an C1-C10-Alkylgruppe, wie z.B. Methyl oder Ethyl, darstellen; R2, R3, R4, R5 vorzugsweise identisch sind und ein Wasserstoffatom darstellen;
- A eine einfache chemische Bindung darstellt, -O-, -S- oder -NH-, vorzugsweise eine einfache chemische Verbindung;
- B Folgendes darstellt:
a) eine mit Cl -C100-Alkylgruppe, unverzweigt oder verzweigt, gesättigt oder nicht, wobei die Alkylkette durch 1 bis 10 Arylgruppen substituiert oder unterbrochen sein kann; oder
b) eine -(CH₂)m-X-Gruppe, in der m eine ganze Zahl zwischen 1 und 100 und X eine mit C1-C100-Alkylgruppe ist, gesättigt oder nicht, die perfluoriert oder teilweise fluoriert sein kann, wobei die Alkylkette durch 1 bis 10 Arylgruppen, die perfluoriert sein können oder nicht, substituiert oder unterbrochen sein kann;
vorzugsweise stellt B eine C9-C20-Alkylgruppe, perfluoriert oder teilweise fluoriert, dar; und
- R1 ein Wasserstoffatom oder eine -OH-, -NH₂-, C1-C100-Alkylgruppe darstellt, die perfluoriert oder teilweise fluoriert sein kann; R1 vorzugsweise eine - OH-Gruppe darstellt,
sowie ihre toxikologisch annehmbaren Salze,
oder auch der folgenden Formel (I'): 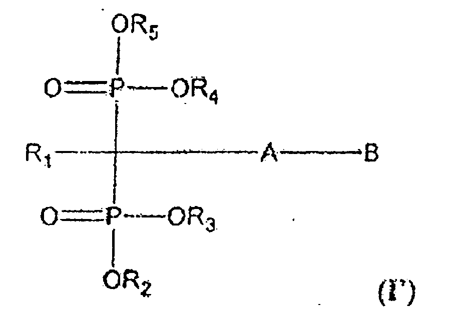 in der:
- B eine -(CH₂)ₘ-D-E-Gruppe darstellt, in der:
- m eine ganze Zahl zwischen 1 und 100 ist,
- D eine einfache chemische Bindung oder eine -O-, -S-, -OCO- -COO-, -COS-, -SCO-, -SCS-, -CONH-, HNCO, HNCO-CF(CF₃)- oder -NH-CO-NH-NH-CS-NH-Gruppe ist, und
- E eine -(O-CF₂-CF₂)ₙ-OR_{f}, -(O-CF₂-CF(CF₃))ₙ-OR_{f}, -(O-CF(CF₃)-CF₂)ₙ-OR_{f}- oder - (O-CF₂-CF₂-CF₂)ₙ-OR_{f}-Gruppe ist, wobei n eine ganze Zahl zwischen 1 und 100 ist und R_{f} eine perfluorierte Cl-C10-Alkylgruppe oder die folgende Gruppe ist: 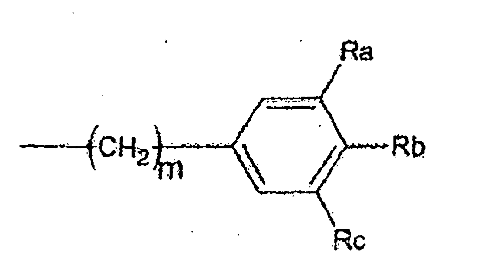 in der Ra, Rb, Rc unabhängig voneinander ein Wasserstoffatom, ein perfluoriertes C1-C100-Alkyl oder eine der folgenden Gruppen darstellt: --(O-CF₂-CF₂)ₙ-OR_{f},- -(O-CF₂-CF(CF₃))ₙ-OR_{f},- (O-CF(CF₃)-CF₂)ₙ-OR_{f} oder - (O-CF₂-CF₂-CF₂)ₙ - OR_{f}, wobei n und R_{f} wie oben definiert sind und m eine ganze Zahl zwischen 0 und 20 ist,
- A Folgendes darstellt:
a) eine einfache chemische Bindung, oder
b) eine -O-, -S- oder -NH-Funktion, und
- R₁, R₂, R₃, R₄ und R₅ wie oben definiert sind, sowie ihre toxikologisch annehmbaren Salze.

6. Verfahren zur Bedeckung nach Anspruch 5, um die Eigenschaften der Benetzbarkeit, der Schmierung oder der Haftung der metallischen oder mineralischen Flächen zu ändern, um die Bildung von Reif oder die Verteilung von Flüssigkeit auf diesen Flächen zu begrenzen, insbesondere durch die Förderung des Epilameffekts, oder um diese Flächen gegen das Anhaften von Graffitis zu schützen oder um das Gleiten von mechanischen Teilen zu erleichtern.

7. Verfahren zur Bedeckung nach einem der Ansprüche 1 bis 4, **dadurch gekennzeichnet, dass** die Gem-Bisphonsäureverbindungen der folgenden Formel (II) entsprechen: 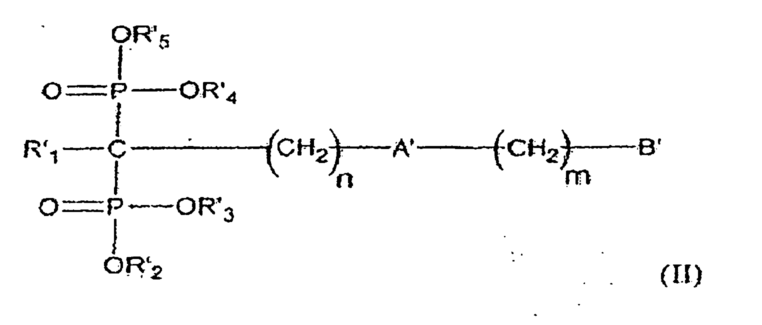 in der:
- n und m ganze Zahlen, voneinander unabhängig zwischen 0 und 20 sind; vorzugsweise n = 3,4 oder 5 und m = 5;
- R'1 ein Wasserstoffatom oder eine -OH-Gruppe, eine Aminosäure oder eine C1-C10-Alkylgruppe darstellt, eventuell fluoriert; R'1 vorzugsweise eine -OH-Gruppe darstellt;
- R'2, R'3, R'4, R'5 unabhängig voneinander ein Wasserstoffatom oder eine C1-C10-Alkylgruppe, wie z.B. Methyl oder Ethyl, darstellen; vorzugsweise R'2, R'3, R'4, R'5 identisch sind und ein Wasserstoffatom darstellen;
- A' eine -L-Y-Gruppe darstellt, wobei:
L eine einfache chemische Bindung oder eine -O-, -S-, - OOC-, -COO-, - CONH-, -HNCO-, -NH-CO-NH-, -NH-, -COS-, -SCO-, -SCS- oder -NH-CS-NH-Gruppe ist;
und
Y ist:
a. eine einfache chemische Bindung;
b. eine Gruppe mit der folgenden Formel: 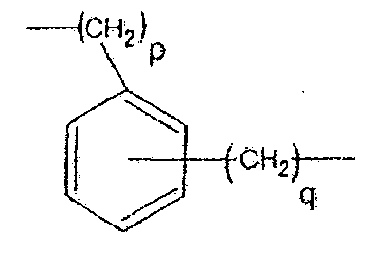 oder
c. eine Gruppe mit der folgenden Formel: 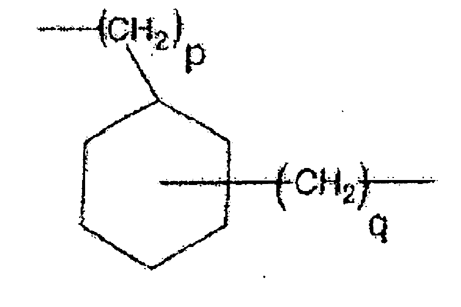 oder
d. eine Gruppe mit der folgenden Formel: 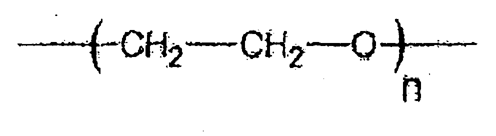 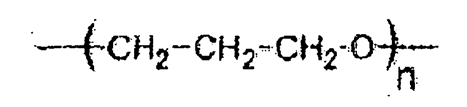
in denen n, p und q unabhängig voneinander ganze Zahlen zwischen 0 und 100 darstellen; und
- B' darstellt:
a) eine Gruppe mit der folgenden Formel: 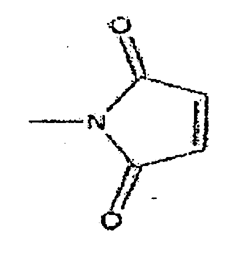 oder
b) eine Gruppe mit der folgenden Formel: 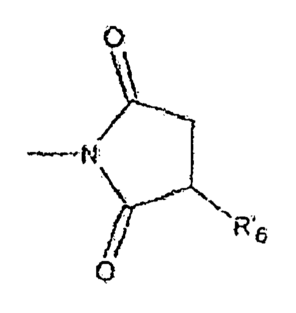 in der R'6 eine Aminosäure, ein lineares oder zyklisches Peptid, insbesondere ein CRGD- oder RGDC-Peptid, ein Protein, ein Glykoprotein, eine einzel- oder doppelstrangige Nukleinsäure, ein Antibiotikum, ein Hormon, ein Saccharid oder Polysaccarid, ein Vitamin, ein Antikörper, ein Fragment eines Antikörpers, ein Hapten, ein Wachstumsfaktor, ein Zytokin oder ein pharmazeutisch aktives Radikal darstellt; vorzugsweise stellt R'6 eine Aminosäure oder ein Peptid dar, wie z.B. CRGD oder RGDC, sowie ihre toxikologisch annehmbaren Salze.

8. Verfahren zur Bedeckung nach Anspruch 7, für die Übertragung von biologisch aktiven Molekülen auf metallische oder mineralische Flächen, um insbesondere die Haftung von Osteoblasten auf orthopädischen Prothesen oder Zahnimplantaten zu erhöhen, um die Mineralisierung zu verbessern, um das Gleichgewicht der osteoblastischen und osteoklastischen Aktivitäten zu ändern oder um Siliziumflächen für die Reinigung, die Haftung oder die Erkennung von biologischen Einheiten zu funktionalisieren.

9. Verfahren zur Bedeckung nach einem der Ansprüche 1 bis 4, **dadurch gekennzeichnet, dass** die Gem-Bisphonsäureverbindungen der folgenden Formel (III) entsprechen: 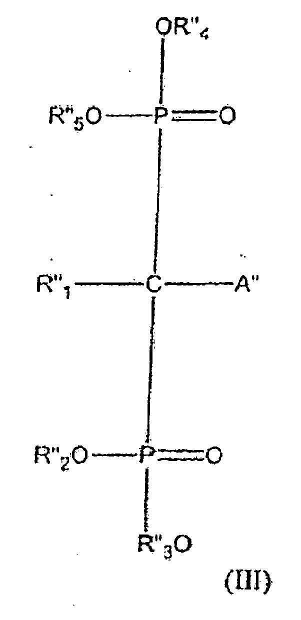 in der:
- R"1 ein Wasserstoffatom, eine -OH, -NH₂-Gruppe, eine C1-C10-Alkylgruppe, eventuell fluoriert, darstellt;
- R"2, R"3, R"4, R"5 unabhängig voneinander ein Wasserstoffatom oder eine C1-C10-Alkylgruppe, wie z.B. Methyl oder Ethyl, darstellen; vorzugsweise R"2, R"3, R"4, R"5 identisch sind und ein Wasserstoffatom darstellen; und
- A" darstellt:
a. eine Gruppe mit der folgenden Formel:
-CH₂O- (CH₂CH₂O)ₙ-X
in der n eine ganze Zahl zwischen 0 und 100 ist und X einen Wasserstoff oder eine C1-C5- Alkylgruppe darstellt; vorzugsweise X eine Methylgruppe darstellt; oder
b. eine Gruppe mit der folgenden Formel: 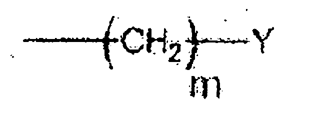
in der m eine ganze Zahl zwischen 0 und 20 ist, vorzugsweise zwischen 3 und 6, und Y eine -NH-CO-CH₃, - CO-NH-CH₃-N(CH₃) -CO-CH₃ oder -CO-N (CH3) 2-Gruppe darstellt,
sowie ihre toxikologisch annehmbaren Salze.

10. Verfahren zur Bedeckung nach Anspruch 9 um die Anlagerung von Makromolekülen, von Mikroorganismen oder Biofilmen auf metallischen oder mineralischen Flächen zu verhindern oder zu begrenzen.

11. Funktionalisiertes Substrat, das durch das Verfahren nach Anspruch 5 erhalten werden kann.

12. Verwendung des funktionalisierten Substrats nach Anspruch 11 in mechanischen Teilen, insbesondere für die Uhrmacherei, die Automobil- oder Luftfahrtindustrie bestimmt, oder auch in elektromechanischen Mikrosystemen oder elektromechanischen Nanosystemen, um die Haftkräfte zu begrenzen, das Gleiten zwischen zwei mechanischen Teilen zu verbessern, die Verteilung von Flüssigkeit auf diesen Flächen zu begrenzen und den Epilameffekt zu fördern oder die Bildung von Reif zu begrenzen.

13. Gem-Bisphonsäureverbindungen, die der folgenden Formel (Ia) entsprechen: 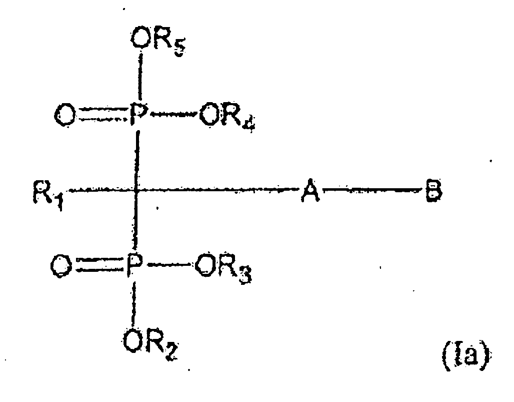 in der: - R2, R3, R4, R5 unabhängig voneinander ein Wasserstoffatom oder eine C1-C10-Alkylgruppe, wie z.B. Methyl oder Ethyl, darstellen; vorzugsweise R2, R3, R4, R5 identisch sind und ein Wasserstoffatom darstellen;
- A eine einfache chemische Bindung, -O-, -S- oder -NH-, darstellt, vorzugsweise eine einfache chemische Bindung;
- B eine-(CH₂)m-X-Gruppe darstellt, in der m eine ganze Zahl zwischen 1 und 100 ist und X eine C1-C100-Alkylgruppe ist, gesättigt oder nicht, die perfluoriert oder teilweise fluoriert sein kann, wobei die Alkylkette durch 1 bis 10 Arylgruppen, die perfluoriert sein können oder nicht, substituiert oder unterbrochen sein kann;
vorzugsweise liegt m zwischen 1 und 20, und X stellt eine C9-C20-Alkylgruppe, perfluoriert oder teilweise fluoriert, dar; noch bevorzugter stellt B eine C9-C20-Alkylgruppe dar, perfluoriert oder teilweise fluoriert; und
- R1 ein Wasserstoffatom oder eine -OH, -NH₂-, C1-C100Alkylgruppe darstellt, die vollständig oder teilweise fluoriert sein kann; R1 vorzugsweise eine - OH-Gruppe darstellt;
sowie ihre toxikologisch annehmbaren Salze.

14. Funktionalisiertes Substrat, das durch das Verfahren nach Anspruch 6 erhalten werden kann.

15. Verwendung des funktionalisierten Substrats nach Anspruch 14 in orthopädischen Prothesen und Zahnimplantaten, um die Haftung der Osteoplasten zu erhöhen, die Mineralisierung zu verbessern oder das Gleichgewicht der osteoblastischen oder osteoklastischen Aktivitäten auf diesen Prothesen oder Implantaten zu ändern.

16. Verwendung des funktionalisierten Substrats nach Anspruch 14 für die Übertragung von biologisch aktiven Molekülen auf metallische oder mineralische Flächen, oder um Flächen für die Reinigung, die Haftung oder die Erkennung von biologischen Einheiten zu funktionalisieren.

17. Gem-Bisphonsäureverbindungen, die der folgenden Formel (IIa) entsprechen: 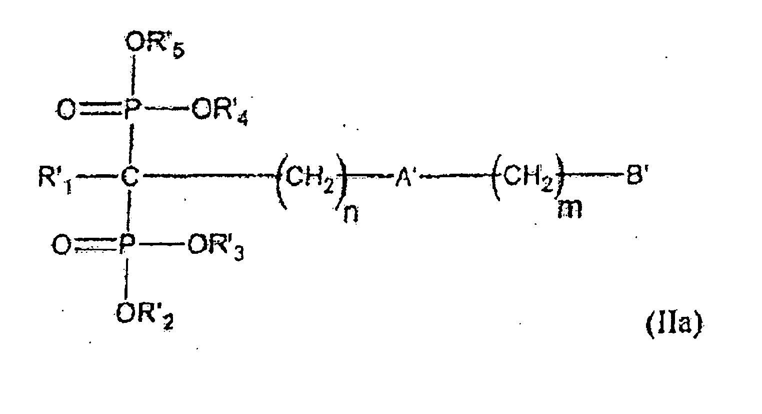 in der:
- n und m ganze Zahlen, voneinander unabhängig zwischen 0 und 20 sind; vorzugsweise n = 3, 4 oder 5 und m = 5;
- R'1 ein Wasserstoffatom, eine -OH-Gruppe, eine Aminosäure, verzweigt oder nicht, oder eine C1-C10-Alkylgruppe darstellt, eventuell fluoriert; vorzugsweise R'1 eine -OH-Gruppe darstellt;
- R'2, R'3, R'4, R'5 unabhängig voneinander ein Wasserstoffatom oder eine C1-C10-Alkylgruppe, wie z.B. Methyl oder Ethyl, darstellen; vorzugsweise R'2, R'3, R'4, R'5 identisch sind und ein Wasserstoffatom darstellen;
- A' eine -L-Y-Gruppe darstellt, wobei:
- L eine einfache chemische Bindung, eine -O-, -S-, - OOC-, -COO-, - CONH-, -HNCO-, -NH-CO-NH-, -NH-Gruppe ist; und
Y ist:
a. eine chemische Bindung;
b. eine Gruppe mit der folgenden Formel: 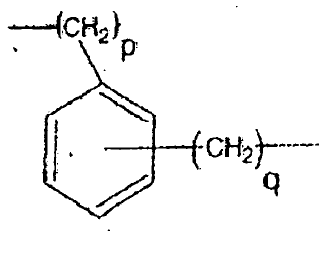 oder
c. eine Gruppe mit der folgenden Formel: 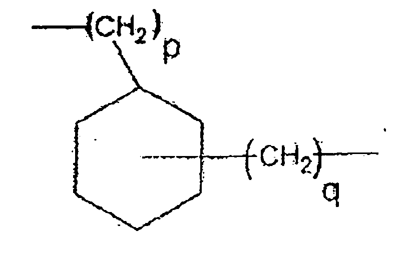 oder
d. eine Gruppe mit der folgenden Formel: 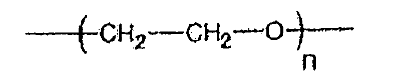 oder 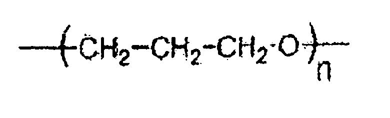 in denen n, p und q unabhängig voneinander ganze Zahlen zwischen 0 und 100 darstellen; vorzugsweise zwischen 0 und 10;
vorzugsweise A' eine -HNCO-Gruppe darstellt; und
B' eine Gruppe mit der folgenden Formel darstellt: 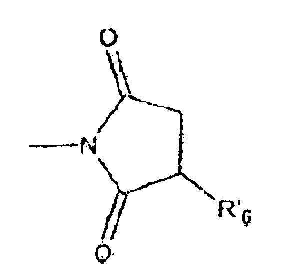 in der R'6 eine Aminosäure, ein lineares oder zyklisches Peptid, insbesondere ein CRGD- oder RGDC-Peptid, ein Protein, ein Glykoprotein, eine einzel- oder doppelstrangige Nukleinsäure, ein Antibiotikum, ein Hormon, ein Saccharid oder Polysaccharid, ein Vitamin, ein Antikörper, ein Fragment eines Antikörpers, ein Hapten, ein Wachstumsfaktor, ein Zytokin oder ein pharmazeutisch aktives Radikal darstellt, vorzugsweise stellt R'6 ein CRGD- oder RGDC-Peptid dar;
sowie ihre toxikologisch annehmbaren Salze.

18. Funktionalisiertes Substrat, das durch das Verfahren nach Anspruch 9 erhalten werden kann.

19. Verwendung der Gem-Bisphonsäureverbindungen mit der Formel (III), wie in Anspruch 9 definiert, gegen die biologische Verschmutzung von metallischen oder mineralischen Flächen oder als Mittel zur biologischen Kompatibilität, insbesondere, um die Anlagerung von Makromolekülen, Mikroorganismen, vorzugsweise Bakterien, oder die Entwicklung des Biofilms auf metallischen oder mineralischen Flächen zu verhindern oder zu begrenzen.

20. Verwendung der Verbindungen mit der Formel (III) nach Anspruch 19, **dadurch gekennzeichnet, dass** die metallischen Flächen zur Gruppe umfassend Eisen, rostfreier Stahl, Chrom, Aluminium, Zink, Titan, Wolfram, Blei oder Kupfer, sowie Oxide, Legierungen oder Zusammensetzungen, enthaltend mindestens eines dieser Metalle, gehören, und die Mineralflächen zur Gruppe umfassend Silizium und seine Derivate, siliziumdioxidhaltige Materialien, Kalziumflächen, keramische oder Zahnflächen, gehören.

21. Verwendung der Verbindungen mit der Formel (III) nach Anspruch 19 oder 20, **dadurch gekennzeichnet, dass** die Flächen die Fläche von Industrie-, Nahrungsmittelindustrie- oder Krankenhausmaterial, von Gebäuden, Konstruktionen oder Land-, Luft- oder Seefahrzeugen, von Klimatisierungs- oder Kühlmaterial oder auch die Fläche von chirurgischen Instrumenten, Prothesen, zahnärztlichen Instrumenten oder biologischen und medizinischen Sensoren sind.
